# EUROPEAN PATENT SPECIFICATION

(11) **EP 3 001 943 B1**
(45) Date of publication and mention of the grant of the patent: **08.11.2017**
(21) Application number: 15187388.2
(22) Date of filing: 29.09.2015
(51) Int. Cl.: A61B 3/10, A61B 3/117, A61B 3/12, A61B 3/15

(54) **OPHTALMIC APPARATUS AND OPHTALMIC APPARATUS CONTROL PROGRAM**
OPHTHALMISCHE VORRICHTUNG UND STEUERUNGSPROGRAMM FÜR OPHTHALMISCHE VORRICHTUNG
APPAREIL OPHTALMIQUE ET PROGRAMME DE COMMANDE D'UN TEL APPAREIL

(30) Priority: 30.09.2014 JP 2014202700
(43) Date of publication of application: 06.04.2016
(73) Proprietor: NIDEK CO., LTD, Gamagori-shi Aichi 443-0038 (JP)
(72) Inventor: ISOGAI, Naoki, Gamagori-shi, Aichi 443-0038 (JP)
(74) Representative: Prüfer & Partner mbB Patentanwälte · Rechtsanwälte

(56) References cited:
- JP-A- 2010 233 998
- JP-A- 2013 154 121
- US-A1- 2012 140 172
- US-A1- 2013 229 623
- US-A1- 2014 204 338

## Description

### BACKGROUND

### Field of the Invention

The present disclosure relates to an ophthalmic apparatus for examining an examinee's eye and a control program used in the ophthalmic apparatus.

### Related Art

One example of an ophthalmic apparatus for examining an examinee's eye is known as an optical coherence tomography device configured to obtain a tomographic image by use of an Optical Coherence Tomography (OCT) using low coherent light (see Patent Document 1). Further, other examples of the ophthalmic apparatus are known as an eye refractive power measuring apparatus, an intraocular pressure measuring apparatus, a fundus camera, a Scanning Laser Ophthalmoscope (SLO), and others.

Conventionally, each of the above ophthalmic apparatuses generally includes a joystick which is operated by an examiner to make alignment of the apparatus with respect to the examinee's eye.

Further, there is known an ophthalmic apparatus for examining an examinee's eye comprising a detection means configured to, in a display means on which the anterior-segment image output by an image output means is displayed, detect a moving direction and a moving amount of one designated point designated on the touch panel when the designated point is moved by the examiner, and a drive control means configured to perform drive control in which, when the detection means detects movement of the designated point, the optometry part is driven in a perpendicular direction to an optical axis of the optometry part based on the moving direction and the moving amount of the designated point detected by the detection means (see Patent Document 2).

### Related Art Documents

### Patent Documents

Patent Document 1: JP-A-2012-213489
Patent Document 2: JP-A-2010-233998

### SUMMARY

### Problems to be Solved by the Invention

Meanwhile, recently proposed as the foregoing ophthalmic apparatus is an apparatus provided with a touch panel and configured to make alignment by operation by an examiner on the touch panel.

However, when alignment of the apparatus is performed by use of the touch panel, the operation on the touch panel is more difficult than the joystick and thus may cause a burden on the examiner.

The present disclosure has been made in view of the circumstances and has a purpose to provide an ophthalmic apparatus easy to operate and an ophthalmic apparatus control program used therein.

### Means of Solving the Problems

To achieve the above purpose, the present disclosure is characterized by the following configuration.

(1) An ophthalmic apparatus for examining an examinee's eye, comprises: an optometry part including an observation optical system configured to observe an anterior segment of the examinee's eye and an examination optical system configured to obtain examination information of the examinee's eye; drive means configured to drive the optometry part in a direction of an examination optical axis with respect to the examinee's eye; drive control means configured to control driving of the drive means; image output means configured to output an anterior-segment image of the examinee's eye photographed by the observation optical system to the display means; and second detection means configured to, in the display means on which the anterior-segment image output by the image output means is displayed, detect at least one of a change in relative distance between and a rotating amount of at least two designated points designated on a touch panel when the designated points are relatively moved, the touch panel being configured to allow designation of any position in an operational region set on a screen of the display means, wherein the drive control means is configured to perform drive control in which, when relative movement of at least the two designated points is detected by the second detection means, the optometry part is driven in a direction of an optical axis based on at least one of the change in relative distance between and the rotating amount of at least the two designated points detected by the second detection means.
(2) An ophthalmic apparatus control program to be used in an ophthalmic apparatus comprising an optometry part including an observation optical system configured to observe an anterior segment of an examinee's eye and an examination optical system configured to obtain examination information of the examinee's eye, and drive means configured to three-dimensionally drive the optometry part with respect to the examinee's eye, wherein the program will be executed by a processor of the ophthalmic apparatus to cause the ophthalmic apparatus to execute: an image output step of outputting an anterior-segment image of the examinee's eye photographed by the observation optical system to display means; a detection step of, in the display means on which the anterior-segment image output in the image output step is displayed, detecting at least one of a change in relative distance between and a rotating amount of at least two designated points designated on a touch panel when the designated points are relatively moved, the touch panel being configured to allow designation of any position in an operational region set on a screen of the display control means; and a drive step of driving the optometry part in a direction of the optical axis based on the change in relative distance between and a the amount of at least the two designated points detected in the detection step when relative movement of at least the two designated points is detected in the detection step.
Further developments of the present invention are given in the dependent claims.

### BRIEF DESCRIPTION OF THE DRAWINGS

FIG. 1 is a schematic external view of a fundus photographing apparatus in an example;
FIG. 2 is a diagram to explain optical systems in the example;
FIG. 3 is a flowchart to explain operations of a touch panel;
FIG. 4 is a flowchart to explain operations of the touch panel;
FIG. 5 is a flowchart to explain operations of the touch panel;
FIGs. 6A and 6B are diagrams to explain operations of the touch panel;
FIG. 7 is a diagram to explain operations of the touch panel;
FIGs. 8A and 8B are examples showing that an anterior segment image imaged by an imaging element is displayed on a display part;
FIGs. 9A and 9B are diagrams to explain alignment detection with respect to an examinee's eye;
FIGs. 10A and 10B are diagrams to explain fine adjustment of alignment in a manual mode;
FIGs. 11A and 11B are diagrams to explain fine adjustment of alignment in an optical axis shift mode; and
FIG. 12 is a diagram to explain a rotating operation on the touch panel.

### DESCRIPTION OF EMBODIMENTS

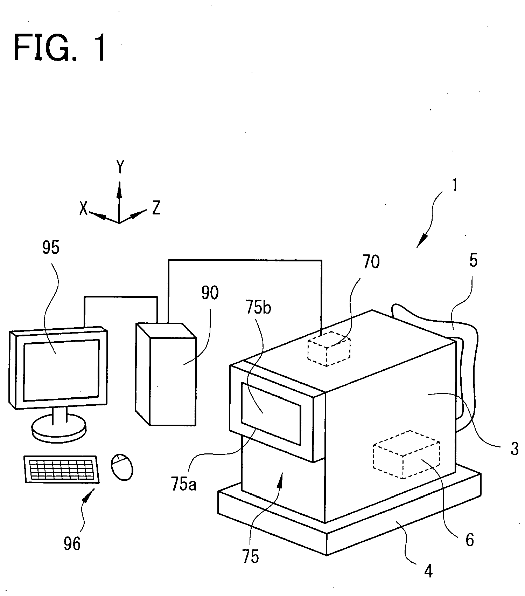

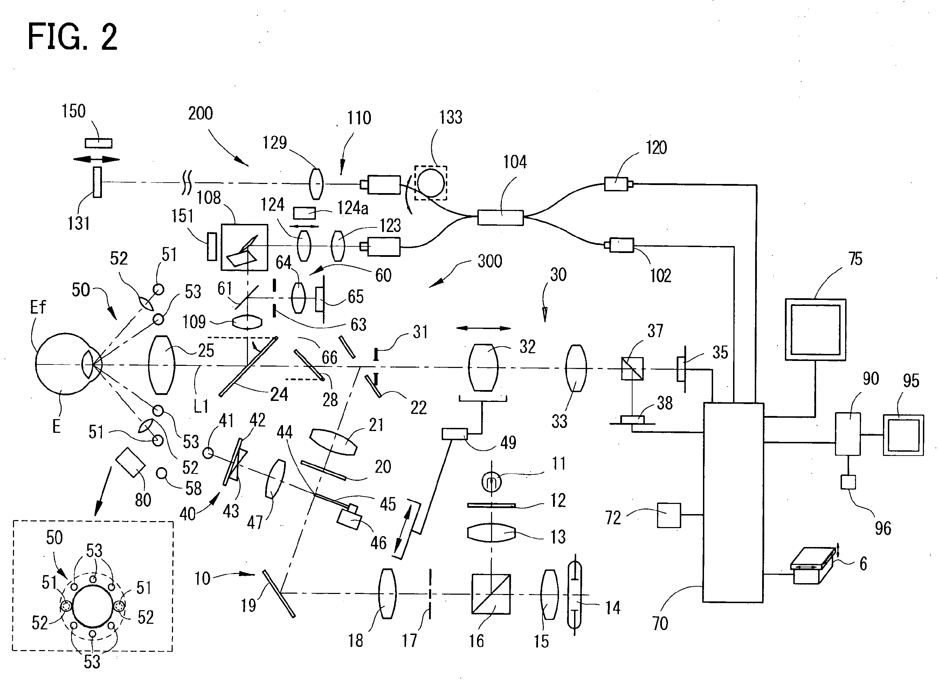

Details of examples of the present disclosure will now be given as below. An ophthalmic apparatus of the present disclosure (e.g., an ophthalmic photographing apparatus 1) in FIG. 1 is an apparatus for examining eyes of an examinee. This ophthalmic apparatus mainly includes for example an optometry part (e.g., a photographing part 3), a drive part (e.g., a XYZ drive part 6, a drive part 124a, a movement mechanism 49, and others), a drive control part (e.g., a control part 70), an image output part (e.g., the control part 70), a first detection part (e.g., the control part 70, a touch panel 75b, and others), and a second detection part (e.g., the control part 70, the touch panel 75b, and others) (see FIG. 2).

The optometry part has an observation optical system (e.g., an anterior-segment observation optical system 60), an examination optical system (e.g., an examination optical system 300, an interference optical system 200, a fundus cameral optical system 30, and others). The observation optical system is configured for example to observe an anterior segment of the examinee's eye. The observation optical system may be arranged for example to photograph the anterior segment of the examinee's eye with infrared light or the like to obtain an anterior-segment image. The observation optical system may also be provided for example with an imaging element (e.g., an imaging element 65). The imaging element may also be placed for example in a position conjugate with the anterior segment of the examinee's eye. The observation optical system may also be configured for example to receive fundus reflection light of the light projected on the examinee's eye and thereby photograph a transillumination image of the examinee's eye.

The examination optical system is configured for example to obtain examination information of the examinee's eye. The examination optical system may also be the interference optical system for photographing a tomographic image of the examinee's eye, a fundus camera for photographing a fundus front image of the examinee's eye, or an SLO. Furthermore, the examination optical system may also be at least one of an eye refractive power measuring apparatus for measuring eye refractive power of the examinee's eye, an intraocular pressure measuring apparatus for measuring intraocular pressure, a visual field measuring apparatus for measuring a visual field, an axial length measuring apparatus for measuring axial length, a corneal shape measuring apparatus for measuring corneal shape, a corneal curvature radius measuring apparatus for measuring curvature radius of a cornea, and others.

The drive part is configured for example to drive the optometry part in three dimensions with respect to an examinee's eye. The drive control part for example controls driving of the drive part.

The image output part is configured for example to output an anterior-segment image photographed by the observation optical system to a display part (e.g., a display part 75). This image output part may also be arranged to output, as an anterior-segment image, a transillumination image photographed by the observation optical system to the display part.

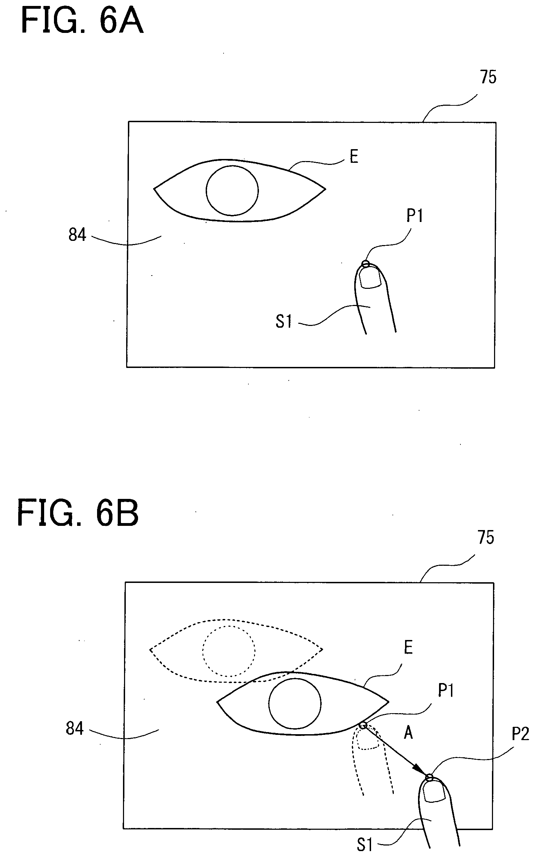

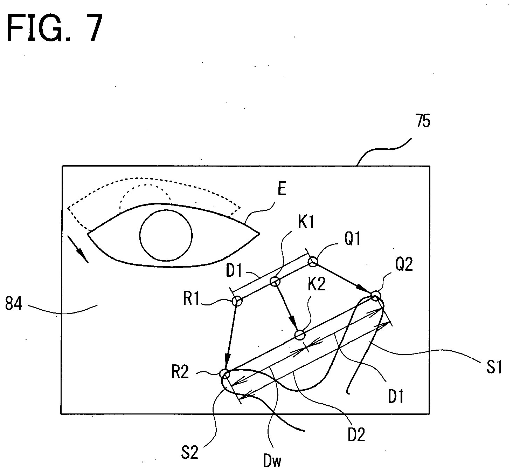

The first detection part detects for example a moving direction and a moving amount or distance of a designated position (e.g., a designated point P1, a designated point P2 (see FIGs. 6A and 6B) and so on) designated on a touch panel (e.g., the touch panel 75b) when the designated point is moved. The touch panel may also be configured to allow designation of any position in an operational region set on a screen of the display part in which an anterior-segment image output by the image output part is displayed. The operational region of the touch panel may be set for example on an image of an anterior segment of an examinee's eye displayed on the display part. The second detection part detects at least one of a change in relative distance between and a rotating amount of at least two designated positions (e.g., a designated point Q1, a designated point Q2, a designated point R1, a designated point R2 (see FIG. 7), and so on) designated on the touch panel when they are relatively moved.

The drive control part may also perform at least one of first drive control and second drive control. The drive control part may concurrently execute for example the first drive control and the second drive control. The first drive control is for example the control in which, when the first detection part detects movement of the designated point, the optometry part is driven in a perpendicular direction to an optical axis of the optometry part based on the moving direction and the moving amount of the designated point detected by the first detection part. Herein, the perpendicular direction to the optical axis may be for example X-Y directions in FIG. 1. The second drive control is for example the control in which, when the second detection part detects relative movement of at least the two designated points, the optometry part is driven in a direction of the optical axis based on at least one of the change in relative distance between and the rotating amount of at least the two designated points detected by the second detection part.

The second detection part may be configured for example to detect a pinch operation on the touch panel. This pinch operation is for example an operation in which two fingers kept touching the touch panel are moved to increase/decrease the distance therebetween. The drive control part may be configured to control the drive part based on a change in relative distance between at least two designated points caused by the pinch operation on the touch panel detected by the second detection part. In this case, for example, the drive control part may be configured to control the drive part to move at least part of the optometry part in an optical axis direction of the optometry part based on the change in relative distance between at least the two designated points. The part of the optometry part may be for example a part of the examination optical system.

The second detection part may also be configured to detect a rotating operation on the touch panel. This rotating operation is for example an operation in which two or more fingers kept touching the touch panel are rotated about an axis corresponding to a perpendicular direction to a screen. The drive control part may also be configured to control the drive part based on a relative rotating amount or angle of at least two designated points based on the rotating operation on the touch panel detected by the second detection part. In this case, for example, the drive control part may also be arranged to control the drive part to move at least part of the optometry part in the optical axis direction of the optometry part based on the rotating amount of at least the two designated points.

The examination optical system may be for example an OCT optical system (e.g., the interference optical system 200) arranged to detect an interference signal generated by interference of the measurement light scanned on the examinee's eye by the scanning means and the reference light to thereby obtain a tomographic image. In this case, the drive part may for example move at least one of a first focusing optical member (the focusing lens 124) of the OCT optical system and a reference mirror (e.g., a reference mirror 131) in the optical axis direction. The drive control part may also control the drive part based on a change in relative distance between and the rotating amount of at least the two designated points detected by the second detection part.

Furthermore, the detection optical system may be for example a front photographing optical system (e.g., the fundus camera optical system 30) configured to receive reflection light from the fundus to obtain a front image of the fundus of the examinee's eye. In this case, the drive part may move a second focusing optical member (e.g., the focusing lens 32) of the front photographing optical system in the optical axis direction. The drive control part may control the drive part based on a change in relative distance between and the rotating amount of at least the two designated points detected by the second detection part.

The ophthalmic apparatus may be configured for example to adjust alignment of the examination optical system with respect to the examinee's eye based on an anterior-segment image photographed by the observation optical system and perform an examination on the examinee's eye.

A processor (e.g., the control part 70) of the ophthalmic apparatus in this example may be configured for example to read in an ophthalmic apparatus control program stored in a storage medium to thereby execute processings such as an image output step, a first detection step, a second detection step, a first drive step, and a second drive step. The image output step is for example a step of outputting an anterior-segment image of an examinee's eye photographed by the observation optical system to the display part. The first detection step is for example a step of, in the display part on which the anterior-segment image output in the image output step is displayed, detecting a moving direction and a moving amount of a designated point designated on the touch panel when the designated point is moved, the touch panel being configured to allow designation of any position in the operational region set on the screen of the display part,.

The second detection step is for example a step of detecting at least one of a change in relative distance between and a rotating amount of at least the two designated points designated on the touch panel when the designated points are relatively moved. The first drive step is for example a step of driving the optometry part in a perpendicular direction to the optical axis of the optometry part based on the moving direction and the moving amount of the designated point detected in the first detection step when movement of the designated point is detected in the first detection step. The second drive step is for example a step of driving the optometry part in the optical axis direction of the optometry part based on the change in relative distance between and the rotating amount of at least the two designated points detected in the second detection step when relative movement of at least the two designated points is detected in the second detection step.

In the present example, a first operation on the anterior-segment image of the examinee's eye and a second operation (different from the first operation) on the anterior-segment image of the examinee's eye are detected and then, based on their detection results, the optometry part is moved up/down, right/left, and back/forth. Since the optometry part is thus caused to move in X-Y-Z directions according to the operations on the anterior-segment image, the alignment operation can be smoothly performed.

### <Examples>

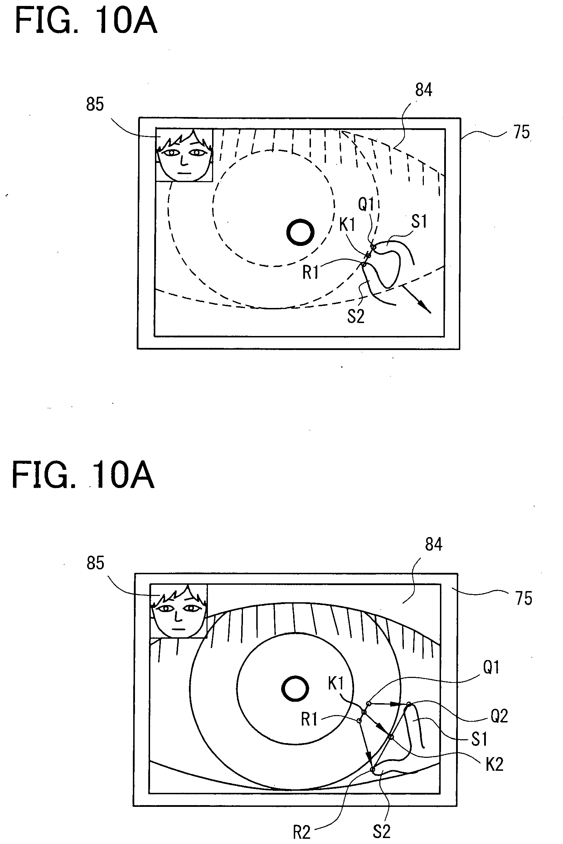

Examples of the present invention will be explained below referring to accompanying drawings. FIGs. 1 to 10A and 10B are diagrams for explanation of the structure of an ophthalmic apparatus in the examples. The examples explained below exemplify the ophthalmic photographing apparatus 1 which is one example of the ophthalmic apparatus. In the examples, the axial direction of an examinee's eye is regarded as a Z direction, the horizontal direction is regarded as an X direction, and a vertical direction is regarded as a Y direction. The surface direction of a fundus may be considered as X-Y directions.

The ophthalmic photographing apparatus 1 in this example mainly includes for example a base 4, the photographing part 3, a face support unit 5, the display part 75 as shown in FIG. 1. The photographing part 3 may contain an examination optical system mentioned later. The photographing part 3 may be provided to be movable with respect to an examinee's eye in three-dimensional directions (X, Y, and Z). The face support unit 5 may be fixed to the base 4 to support the face of an examinee.

The photographing part 3 is relatively moved for example by the XYZ drive part 6 with respect to an examinee's eye in right-left direction, up-down direction (Y direction), and back-front direction.

The display part 75 may be provided for example to the photographing part 3 on an examiner side. The orientation of the display part 75 may be changed arbitrarily by a rotation mechanism. The display part 75 includes a liquid crystal display (hereinafter, it may be simply referred to as an "LCD") 75a and a touch panel (a pointing device) 75b. Of course, the display part 75 is not limited to the structure using the LCD and may be configured to include another display device (an organic EL or the like).

The LCD 75a displays an image of an examinee's eye and information for an examiner. The touch panel 75b is placed on a screen of the LCD 75a. The touch panel 75b in this example is for example a touch panel adapted to a multi-touch operation. For example, a projection capacitance type touch panel may be used. This projection capacitance type touch panel is constituted of for example transparent electrode layers that are placed in a specific pattern and configured to detect the position of a finger based on a change in capacitance between the electrodes caused by approach of a finger thereto. The multi-touch operation is defined as for example an operation to be performed by touching the screen with two or more fingers and simultaneously detecting all input positions at two or more places. The following explanation will be given to the multi-touch operation to be performed by detecting mainly two input positions with two fingers. The multi-touch operation mainly includes a pinch operation (pinch-in operation, pinch-out operation), a rotating operation, a two-finger drag operation, and others. As the touch panel, of course, a touch panel adapted to a single touch operation may be used. The single-touch operation is defined as an operation to be performed by touching a screen (the touch panel 75b) with one finger and detecting an input position at one place.

The display part 75 enables an examiner to input a signal to move the photographing part 3 in each of the X-Y-Z directions to the control part 70 by touch of a finger of the examiner or an input member such as a touch pen. For instance, upon operation on the touch panel 75b, the XYZ drive part 6 is driven to move the photographing part 3 in the X-Y-Z directions. The details of the operation on the touch panel 75b will be described later.

The touch panel 75b may be configured to transmit an operation signal from an examiner to the control part 70 once. In this case, the control part 70 may transmit the operation signal to a host computer 90 mentioned later. For instance, the host computer 90 transmits a control signal corresponding to the operation signal to the control part 70. Upon receipt of the control signal from the host computer 90, for example, the control part 70 may execute various controls based on the control signal.

The ophthalmic photographing apparatus 1 in this example is connected to the host computer (hereinafter, it may be simply referred to as "HC") 90. The HC 90 may be connected to for example a display part 95, an operation part (a keyboard, a mouse, etc.) 96, the control part 70, a detector 120 mentioned later, and others.

### <Optical Systems>

As shown in FIG. 2, the optical systems in this example mainly include an illumination optical system 10 and the examination optical system 300. The examination optical system 300 includes for example the fundus camera optical system 30, the interference optical system (hereinafter, also referred to as an OCT optical system) 200, and others. The fundus camera optical system 30 is used to photograph a fundus (e.g., in a non-mydriatic state) to thereby obtain an infrared fundus image, a color fundus image, and others. The infrared fundus image is used as a fundus observation image for observation of the fundus of the examinee's eye. The OCT optical system 200 non-invasively obtains a tomographic image of the examinee's eye by use of an optical coherence technology. The optical systems may also include a focus target projecting optical system 40, an alignment target projecting optical system 50, and the anterior-segment observation optical system 60.

### <Illumination Optical System>

The illumination optical system 10 includes for example an observation illumination optical system and a photographing illumination optical system. The photographing illumination optical system is mainly provided with a light source 14, a condenser lens 15, a ring slit 17, a relay lens 18, a mirror 19, a black-point plate 20, a relay lens 21, a perforated mirror 22, and an objective lens 25. The photographing light source 14 may be a flash lamp and so on. The black-point plate 20 has a black point at the center.

The observation illumination optical system is mainly provided with a light source 11, an infrared filter 12, a condenser lens 13, a dichroic mirror 16, and an optical system from the ring slit 17 to the objective lens 25. The light source 11 may be for example a halogen lamp or the like. The infrared filter 12 transmits near-infrared light having a wavelength of 750 nm or more, for example. The dichroic mirror 16 is placed for example between the condenser lens 13 and the ring slit 17. The dichroic mirror 16 has a property of for example reflecting light from the light source 11 but transmitting light from the photographing light source 14.

### <Fundus Camera Optical System>

In the fundus camera optical system 30, for example, the objective lens 25, a photographing diaphragm 31, a focusing lens 32, an image-forming lens 33, and an imaging element 35 are arranged. The photographing diaphragm 31 is positioned near an aperture of the perforated mirror 22. The focusing lens 32 is movable in the optical axis direction. The imaging element 35 can be used for example for photographing needing sensitivity in a visible range. The photographing diaphragm 31 is placed for example in a position approximately conjugate with a pupil of the examinee's eye E with respect to the objective lens 25. The focusing lens 32 is moved for example in the optical axis direction by the movement mechanism 49 provided with a motor.

Furthermore, a dichroic mirror 37 having a property of reflecting infrared light and part of visible light but transmitting most part of the visible light is placed between the image-forming lens 33 and the imaging element 35. In a reflection direction of the dichroic mirror 37, an imaging element 38 for observation having sensitivity in an infrared region is placed. Instead of the dichroic mirror 37, a flip-up mirror may be used. This flip-up mirror is for example moved into an optical path at the time of fundus observation and out of the optical path at the time of fundus photographing.

Between the objective lens 25 and the perforated mirror 22, for example, a dichroic mirror (a wavelength selecting mirror) 24 is obliquely placed to be movable in/out as an optical-path splitting member. The dichroic mirror 24 reflects for example wavelength light of the OCT measurement light, and wavelength lights (center wavelength: 940 nm) of the alignment target projecting optical system 50 and the anterior-segment illumination light source 58.

The dichroic mirror 24 has a property of transmitting a wavelength of 900 nm or less including a light source wavelength (center wavelength: 880 nm) of wavelength light of illumination for fundus observation. In sync with photographing by the fundus camera optical system 30, the dichroic mirror 24 is flipped up by the inserting/removing mechanism 66 to retract out of the optical path. The inserting/removing mechanism 66 can be made up of a solenoid, a cam, and others.

Further, in a position adjacent to the dichroic mirror 24, on a side close to the imaging element 35, an optical-path correcting glass 28 is placed to be able to flip up by driving of the inserting/removing mechanism 66. When inserted in the optical path, the optical-path correcting glass 28 serves to correct the position of the optical axis L1 shifted by the dichroic mirror 24.

The light beam emitted from the light source 11 for observation is made into an infrared light beam by the infrared filter 12, and further is reflected by the condenser lens 13 and the dichroic mirror 16, then illuminating the ring slit 17. The light having passed through the ring slit 17 reaches the perforated mirror 22 via the relay lens 18, the mirror 19, the black-point plate 20, and the relay lens 21. The light reflected by the perforated mirror 22 transmits the correcting glass 28 and the dichroic mirror 24, and further is converged once near the pupil of the examinee's eye E by the objective lens 25 and then diffused to illuminate the fundus of the examinee's eye.

The reflection light from the fundus passes through the objective lens 25, the dichroic mirror 24, the correcting glass 28, the aperture of the perforated mirror 22, the photographing diaphragm 31, the focusing lens 32, the image-forming lens 33, and the dichroic mirror 37, and then forms an image on the imaging element 38. The output of the imaging element 38 is input to the control part 70. This control part 70 causes the display part 75 to display a fundus observation image of the examinee's eye imaged by the imaging element 38.

The light beam emitted from the photographing light source 14 passes through the dichroic mirror 16 via the condenser lens 15 and then through the same optical path as the illumination light for fundus observation, so that the fundus is illuminated with visible light. The reflection light from the fundus passes through the objective lens 25, the aperture of the perforated mirror 22, the photographing diaphragm 31, the focusing lens 32, and the image-forming lens 33, then forming an image on the imaging element 35.

### <Focus Target Projecting Optical System>

The focus target projecting optical system 40 is mainly provided with an infrared light source 41, a slit mark plate 42, two deflection prisms 43, a projection lens 47, and a spot mirror 44 obliquely provided in the optical path of the illumination optical system 10. The two deflection prisms 43 are attached to the slit mark plate 42. The spot mirror 44 is obliquely provided in the optical path of the illumination optical system 10. The spot mirror 44 is fixedly attached to a tip of a lever 45. The spot mirror 44 is normally placed in an oblique position to the optical axis and, at a predetermined timing before photographing, it is retracted out of the optical path by rotation of the shaft of a rotary solenoid 46.

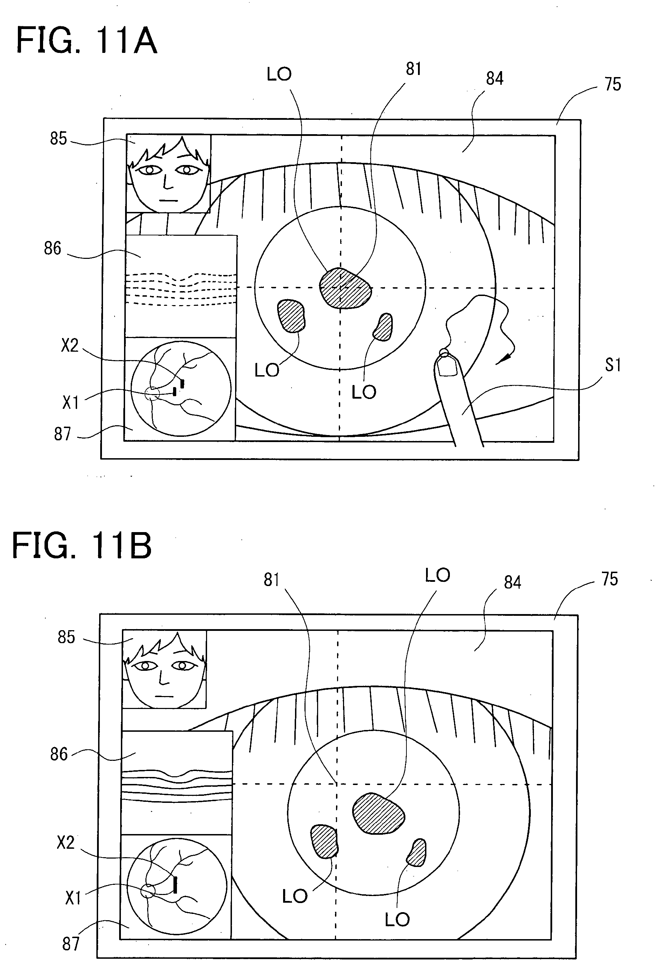

The spot mirror 44 is placed in a position conjugate with the fundus of the examinee's eye E. The light source 41, the slit mark plate 42, the deflection prisms 43, the projection lens 47, the spot mirror 44, and the lever 45 are moved in the optical axis direction by the movement mechanism 49 in sync with movement of the focusing lens 32. The light beam through the slit mark plate 42 of the focus target projecting optical system 40 passes through the deflection prisms 43 and the projection lens 47 and then is reflected by the spot mirror 44 and projected on the fundus of the examinee's eye E via the relay lens 21, the perforated mirror 22, the dichroic mirror 24, and the objective lens 25. When the fundus is out of focus, target images X1 and X2 are projected on the fundus in a separated state according to a displacement direction and a displacement amount (see FIG. 11A). On the other hand, when the fundus is in focus, the target images X1 and X2 are projected on the fundus in a coincident state (see FIG. 11B). The target images X1 and X2 are imaged together with a fundus image by the imaging element 38.

### <Alignment Target Projection Optical System>

The alignment target projection optical system 50 is configured to project an alignment target light beam to the examinee's eye E. In this alignment target projection optical system 50, as illustrated in a lower left dotted frame in FIG. 2, a plurality of infrared light sources are concentrically arranged at intervals of 45 degrees about the photographing optical axis L1. The ophthalmic photographing apparatus in the present example is mainly provided with a first target projection optical system (0° and 180°) and a second target projection optical system.

The first target projection optical system includes infrared light sources 51 and collimating lenses 52. The second target projection optical system includes six infrared light sources 53 arranged in different positions from the first target projection optical system. The infrared light sources 51 are placed in bilaterally symmetric relation with respect to a vertical plane extending along the photographing optical axis L1.

In this case, the first target projection optical system projects infinite targets on a cornea of the examinee's eye E from right and left directions. The second target projection optical system is configured to project finite targets onto the cornea of the examinee's eye E from above and below or from oblique directions. In a main diagram of FIG. 2, the first target projection optical system (0° and 180°) and a part (45° and 135°) of the second target projection optical system are illustrated for convenience.

### <Anterior-segment Observation Optical System>

The anterior-segment observation (photographing) optical system 60 for imaging an anterior segment of the examinee's eye E is mainly provided, on a reflection side of the dichroic mirror 24, with a dichroic mirror 61, a diaphragm 63, a relay lens 64, and a two-dimensional imaging element (a light receiving element; hereinafter, it may be simply referred to as an "imaging element") 65. The imaging element 65 has sensitivity in an infrared region. The imaging element 65 further serves as imaging means for alignment target detection to image the anterior segment illuminated by infrared light emitted from the anterior-segment illumination light source 58 and the alignment targets. Light from the anterior segment illuminated by the anterior-segment illumination light source 58 is received by the imaging element 65 via the objective lens 25, the dichroic mirror 24, and the optical system from the dichroic mirror 61 to the relay lens 64. The alignment light beams emitted from the light sources of the alignment target projection optical system 50 are projected on the cornea of the examinee's eye. The corneal reflection image is received by (projected on) the imaging element 65 via from the objective lens 25 to the relay lens 64.

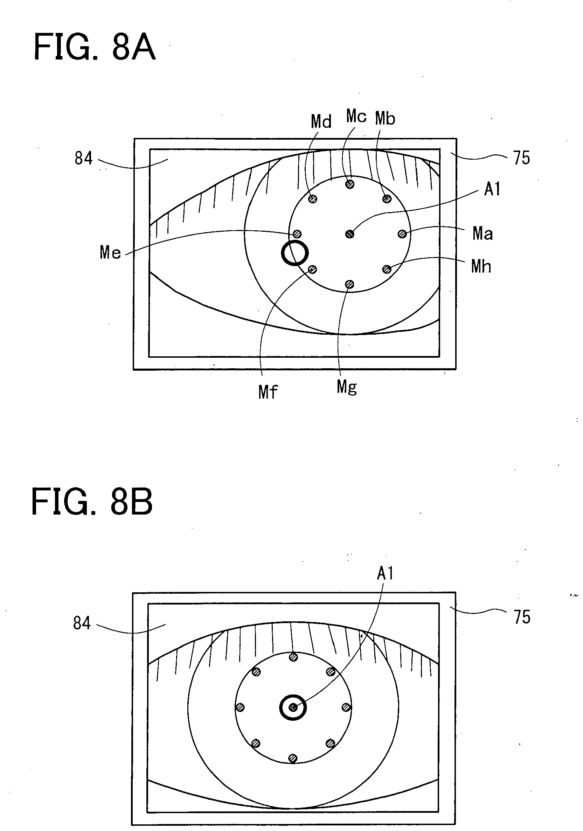

The output of the two-dimensional imaging element 65 is input to the control part 70. The display part 75 displays an anterior-segment image imaged by the two-dimensional imaging element 65 as shown in FIGs. 8A and 8B. The anterior-segment observation optical system 60 is also used as a detection optical system for detecting an alignment state of the apparatus main unit with respect to the examinee's eye.

The anterior-segment observation optical system 60 may be configured to photograph a transillumination image of the examinee's eye. This transillumination image is a pupil internal image photographed by illuminating the inside of a pupil by fundus reflection light, for example.

When the transillumination image is to be photographed, the control part 70 causes the light source 11 functioning as a light source for transillumination image photographing to emit light at a level of light amount enabling photographing of the transillumination image. The light beam from the light source 11 is projected on the fundus. The fundus reflection light illuminates the inside of a crystalline lens of the examinee's eye E and then exits from the pupil. The fundus reflection light exiting from the pupil is reflected by the dichroic mirrors 24 and 61 and received by the imaging element 65. In this case, the dichroic mirrors 24 and 61 and others may be designed with the optical property of reflecting the light of a wavelength emitted from the light source 11. The transillumination image thus photographed contains a shadowed part corresponding to an opacified area due to cataract or other cause.

### <Binocular Observation Optical System>

The present apparatus is provided with a binocular cameral 80 for observation of both eyes of an examinee. As shown in FIG. 2, the binocular camera 80 is provided for example on a side of the photographing part 3 which will face the examinee, and used to photograph the face of the examinee supported with the face support unit 5. A binocular observation image 85 photographed by the binocular camera 80 is displayed on the display part 75 and others by a control part mentioned later (see FIGs. 11A and 11B).

### <OCT Optical System>

Returning to FIG. 2, the OCT optical system 200 has an apparatus structure as a so-called ophthalmic optical coherence tomography (OCT) and is configured to image a tomographic image of the eye E. The OCT optical system 200 is arranged to split the light emitted from a measurement light source 102 into measurement light and reference light by a coupler (a beam splitter) 104. The OCT optical system 200 delivers the measurement light to the fundus Ef of the eye E and also delivers the reference light to the reference optical system 110. The measurement light passes through the collimator lens 123 and the focusing lens 124 and reaches a scanning part 108 in which a reflecting direction of the light is changed by driving of two galvano mirrors, for example. The measurement light reflected by the scanning part 108 is further reflected by the dichroic mirror 24 via the relay lens 109 and then is condensed on the fundus of the examinee's eye via the objective lens 25. Thereafter, the interference light made by synthesis of the measurement light reflected by the fundus Ef and the reference light is received by the detector (the light receiving element) 120.

The detector 120 detects an interference state of the measurement light and the reference light. In the case of Fourier domain OCT, the spectrum intensity of the interference light is detected by the detector 120, and a depth profile (A-scan signal) in a predetermined range is obtained by Fourier conversion with respect to the spectrum intensity data. For example, either spectral-domain OCT (SD-OCT) or swept-source OCT (SS-OC) is adoptable. In the case of the spectral-domain OCT (SD-OCT), for example, a wide-band light source is used as the light source 102 and a light splitter (a spectrometer) is used as the detector 120. In the case of the swept-source OCT, for example, a wavelength changeable light source is used as the light source 102 and a single photodiode is used as the detector 120 (balanced detection may be performed). As another alternative, Time-domain OCT (TD-OCT) may be adopted.

The scanning part 108 is configured to scan the light emitted from the measurement light source on the fundus of the examinee's eye. For example, the scanning part 108 scans the measurement light on the fundus two-dimensionally (in the X and Y directions (transverse direction)). The scanning part 108 is placed in a position approximately conjugate with the pupil. The scanning part 108 is for example constituted of two galvano mirrors arranged at respective reflection angles arbitrarily adjusted by the drive part 151.

Accordingly, the light beam emitted from the light source 102 is changed in its reflection (traveling) direction to scan the fundus in an arbitrary direction. This changes the imaging position on the fundus Ef. Thus, the scanning part 108 has only to be configured to deflect light. For instance, as the scanning part 108, there may be used an acousto-optic modulator (AOM) for changing a traveling (deflection) direction of light, and others, as well as a reflection mirror (galvano mirror, polygon mirror, resonant mirror).

The reference optical system 110 generates reference light to be combined with the reflection light obtained by reflection of the measurement light by the fundus Ef. The reference optical system 110 may be a Michelson optical system or a Mach-Zehnder optical system.

The reference optical system 110 may be configured to move the optical members arranged in the reference optical path to change a difference in optical path length between the measurement light and the reference light. For instance, the reference mirror is moved in the optical axis direction. The configuration for changing the optical path length difference may be placed in the measurement optical path of the measurement optical system.

To be concrete, the reference optical system 110 is mainly provided with for example a collimator lens 129, the reference mirror 131, a reference-mirror drive part 150. The reference-mirror 131 is placed in the reference optical path and configured to be movable in the optical axis to change an optical path length of the reference light. By reflection at the reference mirror 131, the light returns back to the coupler 104 and further is delivered to the detector 120. As another example, the reference optical system 110 is formed of a transmittance optical system (e.g., an optical fiber) and configured to allow the light from the coupler 104 to be delivered to the detector 120 without causing the light to return back to the coupler 104.

### <Control Part>

Next, the control system in the present example will be explained. The control part 70 in this example is connected to the imaging element 65 for anterior-segment observation, the imaging element 38 for infrared fundus observation, the display part 75, each light source, various drive parts, the imaging element 35, a memory 72, and the HC 90. To avoid intricate lines in FIG. 2, lines connecting the imaging element 65, each light source, and various drive parts to the control part 70 are omitted.

The control part 70 causes the display part 75 of the main unit to display an anterior-segment observation image imaged by the imaging element 65 and an infrared fundus observation image imaged by the imaging element 38.

The HC 90 includes a CPU as a processor, a memory (a non-volatile memory) as storage means, and others. The HC 90 is connected to an operation part (e.g., a mouse, a keyboard, etc.) 96, the display part 95, and others. The HC 90 may be configured to transmit a control signal to the control part 70 to control the ophthalmic photographing apparatus 1. The memory of the HC 90 is a non-transient storage medium capable of storing memory content even if power supply is shut off. For instance, a hard disc drive, a flash ROM, a USB memory removably attached to the HC 90, an external server, and others may be used as the memory. The memory stores a photographing control program to control photographing of a front image and a tomographic image by the ophthalmic photographing apparatus 1.

The HC 90 (more concretely, the processor (e.g., CPU) of the HC 90) may obtain a light receiving signal from the detector 120 via the control part 70. The HC 90 may make a calculation processing of the light receiving signal from the detector 120 to generate the tomographic image.

In the case of Fourier-domain OCT, for instance, the HC 90 processes a spectral signal including an interference signal at each wavelength output from the detector 120. By processing of the spectral signal, the HC 90 obtains information of the inside of the examinee's eye (e.g., data of the examinee's eye related to a depth direction (depth information)). To be concrete, the spectral signal (spectral data) is transformed into an equal-interval function I(k) for wave number k (=2π/λ). The HC 90 performs the Fourier conversion of the spectral signal in the wave number k-space to obtain a signal distribution in a depth (Z) region.

### <Touch Panel>

In the present example, the examiner operates the touch panel 75b to move the photographing part 3 and make alignment between the examinee's eye E and the photographing part 3. For instance, the examiner designates or touches a point on the touch panel 75b and drags the designated point, so that the photographing part 3 is moved in the X and Y directions. Further, the examiner performs the pinch operation on the touch panel 75b, so that the photographing part 3 is moved in the Z direction. Herein, the pinch operation is an operation in which two (or more) fingers touch the screen and move away from or toward each other to increase or decrease the distance between the two fingers while they are kept touched on the screen. An operation of decreasing the distance between the two fingers kept touched on the touch panel 75b is called "pinch-in" (or pinch-close) and an operation of increasing the distance between two fingers is called "pinch-out" (or pinch-open).

The touch panel 75b for example has a two-dimensional coordinate (x axis and y axis) set in advance by a program. For instance, the control part 70 recognizes the coordinate of the position (the designated point) designated on the touch panel 75b from a change in electrostatic capacity between electrodes caused when the fingers touch the touch panel 75b.

The memory 72 stores in advance a distance to move the photographing part 3 per unit of coordinate. The control part 70 determines a distance to move the photographing part 3 from differences in x-axis component and y-axis component when the designated point is dragged and calculates a driving amount of the XYZ drive part 6.

### <Control for Touch Panel>

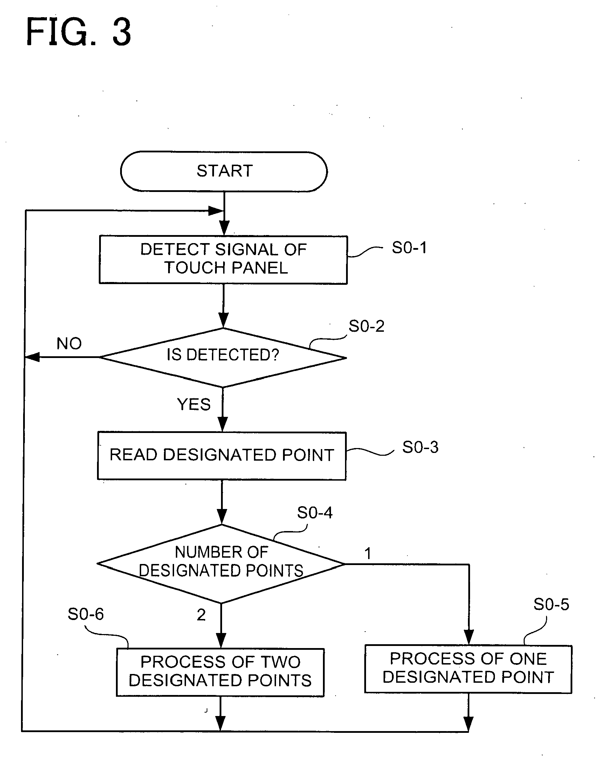

The control for the touch panel 75b will be explained. When the ophthalmic photographing apparatus 1 is started up, the control part 70 executes a main operation step shown in FIG. 3. In the main operation step, the control part 70 reads in signals from the touch panel 75b continuously or at predetermined time intervals (step S0-1), and determines whether a point (designated point) touched with a finger or others on the touch panel 75b is detected (step S0-2). While the touch panel 75b is not pressed, any signal from the touch panel 75b is not detected. Accordingly, the control part 70 determines that no point is designated and returns to step S0-1 to repeatedly receive signals.

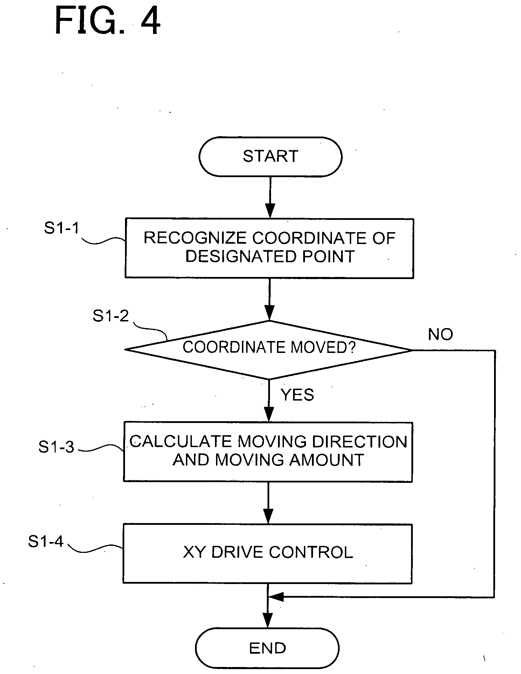

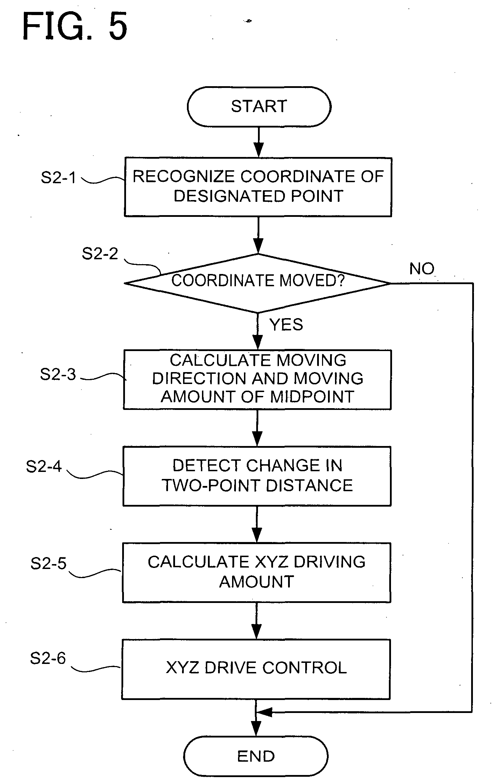

When the control part 70 determines that a point is designated in the step S0-2, the control part 70 obtains the designated point based on a signal from the touch panel 75b (S0-3) and determines the number of designated points (step S0-4). For instance, when the number of designated points is determined as "1", the control part 70 advances to a first operation step shown in FIG. 4 (S0-5). When the number of designated points is 2, the control part 70 advances to a second operation step shown in FIG. 5 (S0-6). The second operation step is a step of detecting the multi-touch operation such as the pinch operation by the examiner.

### <First Operation Step>

The first operation step in FIG. 4 will be first explained. In the first operation step, for example, the control part 70 recognizes the coordinate (the position) of one designated point based on a signal transmitted from the touch panel 75b (step S1-1). In the example of FIG. 6A, the position of a designated point P1 designated with a finger S1 is recognized. While the touch panel 75b is being continuously pressed, a signal output from the touch panel 75b is continuously detected. The control part 70 compares the coordinates based on the signals detected per unit time and determines whether the designated point P1 is moved on the touch panel 75b (step S1-2).

For instance, when a signal detected at certain time t0 is equal to a signal detected at next time t1, the control part 70 determines that the designated point has not been moved. On the other hand, when the signals detected at time t0 and time t1 are different, the control part 70 determines that the designated point has been moved (step S1-2). From a change, or a difference, between the coordinates detected at time t0 and time t1, the moving direction and the moving amount of the dragged designated point are calculated (step S1-3).

For example, as shown in FIG. 6B, the finger S1 is moved in a direction of an arrow A from the designated point P1 to the designated point P2. At that time, the control part 70 detects the coordinate (x0, y0) of the designated point P1 at a first detection time t0 and then detects the coordinate (x1, y1) on the arrow A at a next detection time t1. In this state, since the designated point is moved (dragged) by the finger S1, the control part 70 calculates the moving distance (x1-x0) in the x-axis direction and the moving distance (y1-y0) in the y-axis direction based on the coordinates detected at time t1 and time t0. The moving direction is calculated based on a change from the coordinate (x0, y0) to the coordinate (x1, y1).

When the control part 70 calculates the moving amount and the moving direction of the designated point dragged in step S1-3 as above, the control part 70 controls driving of the XYZ drive part 6 based on the calculated moving amount and moving direction to move the photographing part 3 in the X and Y directions (step S1-4), and then terminates the first operation step.

By the foregoing operations, in sync with dragging of the designated point on the touch panel 75b, the moving amount and the moving direction of the photographing part 3 are controlled and the photographing part 3 is thus moved. Since the photographing part 3 is moved in sync with movement, by dragging, of the anterior-segment image 84 displayed on the display part 75, the examiner is able to make alignment by his/her intuitive operation.

### <Second Operation Step>

The second operation step in FIG. 5 will be explained below. In this second operation step, for instance, the control part 70 recognizes the coordinates (the positions) of two (or more than two) designated points based on signals detected from the touch panel 75b (step S2-1). In the example in FIG. 7, the positions of designated points Q1 and R1 respectively designated with the fingers S1 and S2 are recognized. While the touch panel 75b continues to be pressed, signals from the touch panel 75b are continuously detected. The control part 70 compares the coordinates based on the signals detected per unit time and determines whether the designated points Q1 and R1 have been moved on the touch panel 75b (step S2-2).

For instance, when a signal detected at certain time t0 and a signal detected at next time t1 are equal to each other, the control part 70 judges that the designated points have not been moved. On the other hand, when the signals detected at time t0 and time t1 are different from each other, the control part 70 determines that the designated points have been moved (step S2-2).

When the control part 70 determines that the designated points have not been moved in step S2-2, the control part 70 terminates the second operation step. When determines that the designated points have been moved, the control part 70 executes for example a processing for X-Y driving with reference to a midpoint of the two designated points. For example, the following explanation is given on the assumption that, in a period from the time t0 to the time t1, the designated point Q1 has been moved to the designated point Q2 and the designated point R1 has been moved to the designated point R2 as shown in FIG. 7. In this case, the control part 70 calculates a midpoint K1 between the designated points Q1 and R1 and a midpoint K2 between the designated points Q2 and R2 from the coordinates of those designated points. The control part 70 further calculates the moving direction and the moving amount of the dragged midpoint from a change in the coordinate of the midpoint K2 from the midpoint K1 (step S2-3). The moving direction and the moving amount of the midpoint may be calculated in a similar manner to the method explained in step S1-3.

Furthermore, the control part 70 calculates a distance D1 between the designated points Q1 and R1 and a distance D2 between the designated points Q2 and R2 from the coordinates of those designated points, and calculates a difference Dw between the distance D1 and the distance D2 (step S2-4).

The control part 70 calculates an XYZ driving amount based on the moving direction and the moving amount of the midpoint calculated as above and the degree of the difference Dw (step S2-5).

The control part 70 controls the driving of the XYZ drive part 6 to move the photographing part 3 (step S2-6) based on the XYZ driving amount and the driving direction calculated in step S2-5, and then terminates the second operation step.

The moving amount and the moving direction are repeatedly calculated from changes in the coordinates of the designated points detected at each subsequent detection time (t2, t3, ....). It may be arranged such that the coordinates of the designated points are detected per unit time and, based on the detection result, the moving amount is calculated as above, so that the speed of moving the photographing part 3 can be changed according to the moving speed of the designated points.

### <Control Operation>

Photographing operation of the apparatus configured as above will be explained. As a rough workflow of the present apparatus, auto-alignment or manual alignment is performed and then an OCT image and a color fundus image are photographed. In case the photographed OCT image and others are defective, an optical axis shift mode is implemented to make fine adjustment for alignment and focus.

The flow of photographing will be explained in detail below. Firstly, an examiner asks an examinee to look at a fixation target of a fixation target projecting unit not shown. Then, while observing the binocular observation image 85 including both right and left eyes of the examinee photographed by the binocular camera 80 and displayed on the display part 75, the examiner touches a point on the display part 75 corresponding to the pupil center of one of the right and left eyes. Accordingly, the control part 70 drives the XYZ drive part 6 based on a signal from the touch panel 75b to move the photographing part 3 so as to bring the optical axis L1 to the touched point on the binocular observation image 85. For instance, the control part 70 calculates a driving amount in each direction of the X axis and Y axis from the coordinate of the designated point on the touch panel 75b and drives the XYZ drive part 6 in the X and Y directions. After completion of moving the photographing part 3 in the X and Y directions, the control part 70 may move forward the photographing part 3 until an alignment bright point is detected.

When the anterior-segment observation image of the examinee's eye is obtained by the observation optical system 60 in the above manner, the anterior-segment observation image 84 appears on the display part 75. In this case, as shown in FIGs. 8A and 8B, eight target images (first alignment target images) Ma to Mh come to appear. In this case, an imaging area by the imaging element 65 is preferably determined as such a range including pupil, iris, and eyelashes of the anterior segment at the time of alignment completion.

### <Auto-alignment>

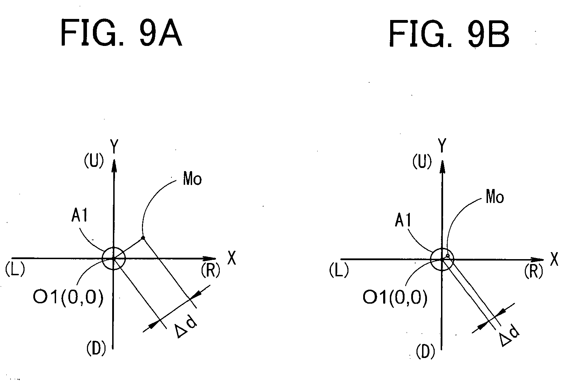

When the alignment target images Ma to Mh are detected by the two-dimensional imaging element 65, the control part 70 starts auto-alignment control. The control part 70 detects an alignment deviation amount Δd of the photographing part 3 with respect to the examinee's eye based on an imaging signal output from the two-dimensional imaging element 65. To be more concrete, the control part 70 detects, as an approximate corneal center, an XY coordinate of the center of the ring shape defined by the target images Ma to Mh projected in a ring form, and calculates the deviation amount Δd between an alignment reference position O1 (e.g., an intersection between an imaging plane of the imaging element 65 and the photographing optical axis L1) in the X and Y directions set on the imaging element 65 in advance and the corneal center coordinate Mo (see FIGs. 9A and 9B). It may be arranged to detect the pupil center by image processing and detect misalignment from the deviation amount between the coordinate position of the detected pupil center and the reference position O1.

The control part 70 further operates the auto-alignment by control driving of the XYZ drive part 6 so that the deviation amount Δd falls within an allowable range A1 of alignment completion. It is determined whether the alignment in the X and Y directions is proper according to whether the deviation amount Δd falls within the allowable range A1 of alignment completion and this state continues for a predetermined time (e.g., a period of time corresponding to 10 frames of image processing, a period of time of 0.3 seconds, and others) (whether the alignment condition is satisfied).

The control part 70 compares the interval between the infinite target images Ma and Me and the interval between the finite target images Mh and Mf, which are detected as above, to calculate an alignment deviation amount in the Z direction. In this case, the control part 70 calculates an alignment deviation amount in a working distance direction with respect to the examinee's eye by utilizing the characteristics that the image interval of the target images Mh and Mf changes whereas the interval of the foregoing infinite target images Ma and Me hardly changes when the photographing part 3 is displaced in the working distance direction (for the details, see JP-A-6(1994)-46999).

Regarding the Z direction, similarly, the control part 70 calculates the deviation amount from the alignment reference position in the Z direction, and operates the auto-alignment by driving control of the XYZ drive part 6 so that the deviation amount falls within the alignment allowable range regarded as representing alignment completion. It is then determined whether the alignment in the Z direction is proper according to whether the deviation amount in the Z direction is in the alignment completion allowable range for a predetermined time (whether the alignment condition is satisfied).

When the alignment state in the X-Y-Z directions meets the alignment completion condition through the foregoing alignment operations, the control part 70 judges the alignment in the X-Y-Z directions is achieved and then shits the flow to a next step.

Herein, when the alignment deviation amount Δd in the X-Y-Z directions falls within the allowable range A1, the control part 70 stops driving of the drive part 6 and outputs an alignment completion signal. Even after alignment completion, the control part 70 detects the deviation amount Δd as needed and, if the deviation amount Δd goes beyond the allowable range A1, the control part 70 starts the auto-alignment again. In other words, the control part 70 performs the control (tracking) of causing the photographing part 3 to track the examinee's eye so that the deviation amount Δd satisfies the allowable range A1.

In the case of photographing a diseased eye, the foregoing auto-alignment may be failed. For instance, there may be a case where a corneal center or a pupil center and others could not be detected or stable alignment could not be achieved due to cataract, drooping eyelid, eye nystagmus, fixation failure, or another cause. In this case, the control part 70 determines that the alignment in the X-Y-Z directions is not proper and switches the alignment mode to a manual mode which will be explained below. The manual mode may be switched automatically from the auto mode by determination of the control part 70 or by an operation of the examiner.

### <Manual Alignment>

In the manual mode, the examiner manually makes alignment of the photographing part 3 based on the aforementioned operation on the touch panel 75b. Accordingly, even for a diseased eye for which auto-alignment is difficult, alignment can be achieved by the operation of the examiner.

When the alignment mode is switched to the manual mode, the control part 70 adjusts the alignment between the examinee's eye E and the photographing part 3 based on the operation on the touch panel 75b explained in FIGs. 3 to 7. In this example, based on observation of the anterior-segment image 84 photographed by the observation optical system 60, the alignment of the photographing part 3 with respect to the anterior segment is adjusted.

For example, the following explanation is given on the assumption that, as shown in FIG. 10A, the anterior-segment image 84 is displayed with the pupil of the examinee's eye displaced to the upper left in an anterior-segment displaying region and in a blurred state because the pupil is located farther than the working distance of the photographing part 3.

For instance, the touch panel 75b is operated with two fingers (a forefinger S1 and a thumb S2) to adjust alignment with respect to the anterior segment. The examiner touches arbitrary points on the anterior-segment image 84 with the forefinger S1 and the thumb S2, increases the interval between the two fingers S1 and S2 for pinch-out, and further drags the both fingers to the lower right. At that time, the designated point of the forefinger S1 and the designated point of the thumb S2 before dragging are respectively referred to as a designated point Q and a designated point R1 and the designated points after dragging are referred to as a designated point Q2 and a designated point R2. The control part 70 finds the coordinates of the a midpoint K1 between the designated points Q1 and R1 and a midpoint K2 between the designated points Q2 and R2 and calculates the moving amount and the moving direction from the midpoint K1 to the midpoint K2. Furthermore, the control part 70 calculates the difference Dw as mentioned above. The control part 70 controls the XYZ drive part 6 based on the calculated moving amount, moving direction, and difference Dw, to three-dimensionally move the photographing part 3 to the front upper left toward the examinee's eye. When the photographing part 3 is moved to the front upper left, the anterior-segment image 84 displayed on the display part 75 becomes clear at the pupil center and others as shown in FIG. 10B. If the photographing part 3 is located nearer to the examinee's eye than the working distance, the examiner may move the photographing part 3 toward the near side of the examinee by the pinch-in operation.

The examiner appropriately performs the drag operation and the pinch operation while observing the anterior-segment image 84 to thereby move the photographing part 3 to an appropriate position with respect to the examinee's eye. Since the drag operation and the pinch operation are concurrently performed as above to three-dimensionally move the photographing part 3, the manual alignment can be carried out more smoothly. Further, the drag operation and the pinch operation can be easily conducted with one hand as described above, so that the examiner can open the eyelids of the examinee's eye with the other hand during alignment adjustment in the X-Y-Z directions. Of course, the drag operation and the pinch operation may be performed separately.

The touch panel operation for alignment in the X-Y-Z directions can be performed in any position on the display part 75. This can save a step of checking the position of a movement button on the display part 75 at each operation as compared with the case where the photographing part 3 is moved by operation of a movement button and others displayed on the display part 75.

### <Detection of Focus State / Auto-focus>

Upon completion of the alignment performed automatically or manually as explained above, the display part 75 displays thereon a live image of a fundus captured by the imaging element 38. After completion of the alignment, furthermore, the control part 70 makes an auto-focus operation with respect to the fundus of the examinee's eye. For instance, the fundus observation image 87 captured by the imaging element 38 includes, at the center of the fundus image, the focus target images X1 and X2 projected by the focus target optical system 40 (see FIGs. 11A and 11B). Herein, the focus target images X1 and X2 are projected in separate positions during out-of-focus and in a coincident position during in-focus. The control part 70 detects the target images X1 and X2 by image processing and obtains their separation information. Based on the separation information of the target images X1 and X2, the control part 70 then controls driving of the movement mechanism 49 to move the lens 32 to focus on the fundus.

### <Optimization Control>

After outputting the alignment completion signal, the control part 70 generates a trigger signal to start the optimization control and starts an operation for optimization control. By this optimization, the control part 70 enables the examiner to observe a desired fundus portion with high sensitivity and high resolution. In this example, the optimization control is the control for optical path length adjustment, focus adjustment, and adjustment of polarized state (polarizer adjustment). The optimization control has only to meet a predetermined allowable condition with respect to the fundus and does not necessarily need to bring the adjustments into the most appropriate states.

In the optimization control, the control part 70 performs initialization control to set the positions of the reference mirror 131 and the focusing lens 124 as initial positions. After completion of the initialization, the control part 70 moves the reference mirror 131 in one direction from the set initial position at a predetermined step to perform first optical-path length adjustment (First automatic optical-path-length adjustment). Concurrently with the first optical-path length adjustment, the control part 70 obtains in-focus position information (e.g., the moving amount of the lens 32) with respect to the fundus of the examinee's eye based on a focus result of the fundus camera optical system 30 with respect to the fundus of the examinee's eye. When obtains the in-focus position information, the control part 70 moves the focusing lens 124 to the in-focus position for auto-focus adjustment (focus adjustment). It is to be noted that the in-focus position is required to be a position enabling acquisition of contrast of the tomographic image 86 acceptable as an observation image and does not need to be an optimal position of the focus state.

After completion of the focus adjustment, the control part 70 moves the reference mirror 131 in the optical axis direction and performs second optical-path-length adjustment of re-adjusting the optical path length (fine adjustment of the optical path length). After completion of the second optical-path-length adjustment, the control part 70 drives the polarizer 133 serving to adjust a polarized state of the reference light to adjust a polarized state of the measurement light (for the details, see JP-A-2012-56292).

When the optimization control is completed, the examiner is allowed to observe a desired fundus portion with high sensitivity and high resolution.

However, in case of a diseased eye, a good fundus image may not be obtained even after completion of the optimization control. For example, the image may be darkened and vignetting or flare reflection may be caused due to cataract, drooping eyelid, and others. In this case, the examiner judges the image to be defective and thus touches a point on the anterior-segment observation image 84 displaying the pupil center of the anterior segment. When the point corresponding to the pupil center is touched, the control part 70 establishes for example an optical axis shift mode (a second manual mode). This optical axis shift mode is a mode of finely or minutely adjusting the alignment of the photographing part 3 by manual operation in order to shift the photographing optical axis L1 of the photographing part 3 within the pupil of the examinee's eye (see FIGs. 11A and 11B). For instance, the examiner can shift the photographing optical axis L1 within the pupil to avoid an opaque portion of cataract, eyelids, and others, so that even a diseased eye can be photographed in a less defective image.

When the control part 70 establishes the optical axis shift mode, the control part 70 moves the photographing part 3 for example based on a touch operation on the anterior-segment image 84. At that time, the control part 70 may display the anterior-segment image 84 or its pupil portion in an enlarged size on the display part 75 to make it easy to observe the inside of the pupil. The control part 70 may display a transillumination image of the examinee's eye on the display part 75. Since the transillumination image of the examinee's eye includes an opaque area due to cataract and others photographed as shadow, the examiner can recognize the position of an opacified portion LO and make fine adjustment of alignment to displace the opacified portion LO from the photographing optical axis L1.

In the examples shown in FIGs. 11A and 11B, the control part 70 displays a cross mark 81 and others to indicate the photographing optical axis L1 on the anterior-segment image. In this case, the examiner makes fine adjustment of the positional relationship between the cross mark 81 appearing on the anterior-segment image 84 and the pupil by the drag operation on the touch panel 75b. For instance, the examiner touches any point on the anterior-segment image 84 as shown in FIG. 11A and performs the drag operation. The control part 70 controls driving of the XYZ drive part 6 in a similar manner to the explanation in FIGs. 3 to 5 based on an operation signal from the touch panel 75b to move the photographing part 3 in the X and Y directions with respect to the examinee's eye E. In association with movement of the photographing part 3, the position of the pupil displayed on the display part 75 is moved, thus resulting in a change in the relative positional relationship between the pupil and the cross mark 81 (see FIG. 11B). Accordingly, the examiner performs the drag operation so that the cross mark 81 searches within the pupil to make fine adjustment of the positional relationship between the cross mark 81 and the pupil. The control part 70 moves the photographing part 3 with respect to the eye E so that the photographing optical axis L1 searches within the pupil of the examinee's eye E.

The examiner performs the drag operation on the touch panel 75b to move the photographing optical axis L1 within the pupil and simultaneously checks the tomographic image 86 displayed on the display part 75, and continues to perform the drag operation until the tomographic image 86 appears clearly. In this manner, the examiner makes fine adjustment of alignment between the photographing part 3 and the examinee's eye E.

Furthermore, the examiner can perform the multi-touch operation on the touch panel 75b to adjust the examination optical system 300 in the Z direction. For instance, the examiner may perform the pinch operation to make focus adjustment of the interference optical system 200. In this case, the control part 70 may drive the drive part 124a based on an operation signal from the touch panel 75b generated by the pinch operation by the examiner to move the focusing lens in the optical axis direction. For instance, the control part 70 may move the focusing lens 124 in a direction to come close to the examinee's eye when the examiner performs the pinch-out operation and reversely move the focusing lens 124 in a direction to come away from the examinee's eye when the examiner performs the pinch-in operation. The examiner may repeat the pinch operation until the tomographic image 86 appears clearly while observing the tomographic image 86 displayed on the display part 75.

The examiner may concurrently perform the drag operation and the pinch operation described above to make fine adjustment of alignment of the photographing part 3 in the X and Y directions and fine adjustment of focus of the interference optical system 200 in a parallel way. In this case, the control part 70 may concurrently drive the XYZ drive part 6, the drive part 124a, and others based on operation signals from the touch panel 75b generated by the drag operation and the pinch operation by the examiner.

By continuing the fine adjustment of the positional relationship between the photographing optical axis L1 and the examinee's eye until the tomographic image 86 appears with high photographing sensitivity as above, for example, the photographing optical axis L1 is moved out of a diseased portion (e.g., an opacified portion of a crystalline lens), so that even a diseased eye can be photographed in a good image.

Since an eyeball is a spherical object, when the photographing optical axis L1 is moved in the X and Y directions, the alignment in the Z axis direction may be displaced. Accordingly, if the alignment operation in the X and Y directions and the alignment in the Z axis direction could not be performed concurrently, the alignment operation in the Z direction has to be performed by switching every after the alignment in the X and Y directions is adjusted. Since the alignment in the X and Y directions and the alignment in the Z direction can be adjusted by a series of operations as mentioned above, some steps related to the operation by the examinee can be reduced.

Furthermore, the examiner can intuitively make fine adjustment of alignment and focus by the drag operation and the pinch operation on the touch panel 75b, and thus can naturally make alignment adjustment.

Upon completion of the fine adjustment of focus of the photographing optical axis L1 and the interference optical system 200, the examiner performs an operation to start photographing. The operation to start photographing may be carried out, for example, by single tapping, double tapping, or tapping with two or more fingers on the touch panel 75b or by an action of separating the finger or fingers from the touch panel 75b. When the tapping is carried out by the examiner or when any signal from the touch panel 75b does not get detected, the control part 70 may start photographing of the tomographic image 86. The tap operation is for example an operation of lightly pressing (tapping) a touch panel, that is, single tapping is an operation of lightly pressing once and double tapping is an operation of lightly pressing twice.

When obtains the signal to start photographing, the control part 70 controls driving of the scanning part 108 to scan the measurement light on the fundus. The HC 90 creates the tomographic image 86 as a still image based on a detection signal from the detector 120. The HC 90 stores the tomographic image 86 in the memory 72.

Subsequently, the control part 70 shifts the flow to the step of obtaining a color fundus image by the fundus camera optical system 100. Firstly, the control part 70 turns off an internal fixation lamp not shown and a fundus illumination light source (e.g., the light source 11). The control part 70 then drives the inserting/removing mechanism 66 to move the dichroic mirror 24 out of the optical path and cause the photographing light source 14 to emit light.

When the photographing light source 14 emits light, the fundus of the examinee's eye is illuminated with visible light. The reflection light from the fundus passes through the objective lens 25, the aperture of the perforated mirror 22, the photographing diaphragm 31, the focusing lens 32, the image-forming lens 33, and the dichroic mirror 37 and then forms an image on the two-dimensional imaging element 35. The color fundus image photographed by the two-dimensional imaging element 35 is received by the HC 90 and thereafter stored in the memory 72.

In the foregoing explanation, the control part 70 adjusts the focus of the interference optical system 200 based on the operation signal from the touch panel 75b generated by the pinch operation by the examiner, but the present disclosure is not limited thereto. For instance, the control part 70 may make focus adjustment of the fundus observation image 87 photographed by the fundus camera optical system 30 based on the operation signal from the touch panel 75b. For example, when the control part 70 detects the pinch operation by the examiner, the control part 70 may control driving of the movement mechanism 49 to move the focusing lens 32 of the fundus camera optical system 30. A method of driving the focusing lens 32 based on the pinch operation may be similar to that for the aforementioned interference optical system 200.

Furthermore, the control part 70 may adjust the optical path length of the interference optical system 200 by the pinch operation by the examiner. In this case, the control part 70 may adjust the optical path length of the reference optical system 110 by driving the drive part 150 to move the reference mirror 131 in the optical axis direction based on the operation signal from the touch panel.

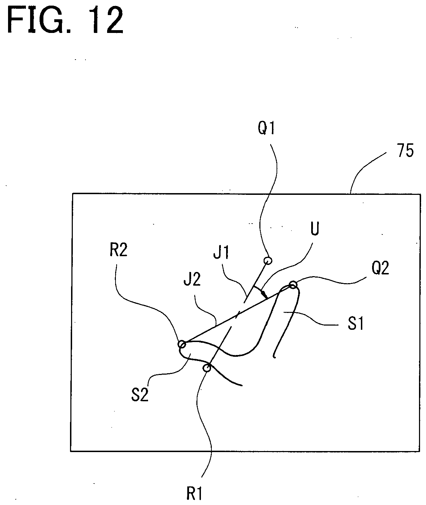

In the foregoing explanation, the examiner adjusts alignment of the photographing part 3 in the X-Y-Z directions and focus of the interference optical system 200 by the drag operation and the pinch operation, but the present disclosure is not limited thereto. For instance, the examiner may adjust alignment in the X-Y-Z directions and hence focus of the interference optical system 200, and others by the drag operation and the rotating operation on the touch panel 75b. Herein, the rotating operation is an operation in which two fingers kept touching a screen are rotated about a perpendicular direction to the screen. For instance, the control part 70 may perform various adjustments based on a rotating amount by the rotating operation performed by the examiner. The rotating amount may be calculated by an angle U and others formed between a line J1 connecting the designated points Q1 and R1 before movement defined by touching of the two fingers S1 and S2 and a line J2 connecting the designated points Q2 and R2 after movement, as shown in FIG. 12.

The control part 70 may adjust at least one of various settings of the apparatus, the photographing light quantity, optimization of the interference optical system 200 (e.g., focus, optical path length, polarizer, and so on), focus of the fundus cameral optical system 30 by detecting the signals based on the pinch operation, rotating operation, and others on the touch panel.

When a clockwise rotating operation is performed by the examiner, for instance, the control part 70 may control driving of the drive part 124a based on the rotating operation signal related to clockwise rotation output from the touch panel 75b to move the focusing lens 124 of the interference optical system 120 in a direction to come close to the examinee in the optical axis direction. Similarly, when a counterclockwise rotating operation is performed by the examiner, the control part 70 may control driving of the drive part 124a based on the rotating operation signal related to counterclockwise rotation output from the touch panel 75b to move the focusing lens 124 of the interference optical system 200 in a direction to come away from the examinee in the optical axis direction. In this manner, the control part 70 may drive the XYZ drive part 6, the drive part 124a, the movement mechanism 49, and others in the Z direction based on the operation signals from the touch panel 75b by the multi-touch operation such as the pinch operation and the rotating operation.

In the above-mentioned explanation, the examiner performs the drag operation and the touch operation such as the pinch operation on the touch panel 75b provided in the ophthalmic photographing apparatus 1 to control driving of each drive part in the Z direction, but the present disclosure is not limited thereto. For instance, alignment of the ophthalmic photographing apparatus 1 in the X-Y-Z directions, optimization of the interference optical system, focus adjustment of the front photographing optical system, and others may be controlled remotely by operation of a pointing device provided to an external terminal connected to the ophthalmic photographing apparatus 1 with or without wires. Herein, the external terminal may include for example a tablet computer and a personal computer.

Fine adjustment of alignment of the photographing optical axis L1 in the X and Y directions with respect to the examinee's eye E is not necessarily made by the drag operation with two fingers as shown in FIGs. 10A and 10B, and may be made by the drag operation with one finger. In the case of performing fine adjustment in the Z direction, the examiner may also further touch the touch panel 75b with another finger to conduct the pinch operation. In this case, the control part 70 controls the XYZ drive part 6, the drive part 124a, the movement mechanism 49, and others based on the drag operation, the pinch operation, and others on the touch panel 75b, and make fine adjustment of alignment, focus, and others of the examination optical system 300.

The fine adjustment of alignment of the photographing optical axis L1 is not necessarily made by the drag operation, and may be made for example by the touch operation. For instance, the control part 70 may drive the XYZ drive part 6 in the X and Y directions to bring the photographing optical axis to the position of the designated point on the anterior-segment image designated by the examiner.

In the foregoing explanation, the control part 70 is configured to perform alignment, optimization, and others of the examination optical system 300 based on the touch operation on the anterior-segment image displayed on the display part 75, but the present disclosure is not limited thereto. For instance, the control part 70 may be configured to perform alignment, optimization, and others of the examination optical system 300 based on the touch operation on an operational region set at the position outside the displayed anterior-segment image 84 on the screen of the display part 75.

The display part 75 explained in this disclosure is configured to display thereon the anterior-segment image 84 in real time; however, it may also be configured to display a still image. In this case, the examiner performs the touch operation on the still image of an anterior segment. The control part 70 may also be configured to displace the optical axis by a displacement amount of alignment center from the position designated by the touch operation applied on the still image. Further, the control part 70 may also be configured to control the XYZ drive part 6 and others to displace the optical axis by the displacement amount to perform alignment of the photographing part 3 in the X-Y-Z directions.

When the touch operation is performed on the anterior-segment still image, the control part 70 may perform auto-alignment with respect to the touched point on the still image. Specifically, the control part 70 may switch a reference position for auto-alignment to a designated point designated on the still image.

The alignment mode may be switched from the auto mode to the manual mode when the examiner touches the anterior-segment image 84. For instance, when the anterior-segment alignment is to be adjusted in the auto-alignment mode, if the anterior segment of the examinee does not appear on the anterior-segment image 84 and the auto-alignment is obviously failed, the examiner also may touch at least one of the binocular observation image 85 and the anterior-segment image 84 to switch between the auto mode and manual mode. The control part 70 may switch the alignment mode to the manual mode when the examiner touches the touch panel with his/her hand and switch the alignment mode to the auto mode when the examiner separates his/her hand from the touch panel.

In the above explanation, the control part 70 is configured to move the focusing lens 32 based on the pinch operation by the examiner to adjust focus of the fundus camera optical system 30, but the present disclosure is not limited thereto. For instance, the drive part for driving the imaging element 38 of the fundus camera optical system 30 and others in the optical axis direction may be provided, so that the control part 70 controls driving of the drive part to drive the imaging element 38 in the optical axis direction to adjust focus of the fundus camera optical system 30. The focus can be changed largely if only the imaging element 38 is more slightly moved as compared with the case where the focusing lens 32 is moved. This contributes to downsizing of the apparatus.

In the above explanation, when fine adjustment of alignment of the photographing part 3 in the X-Y-Z directions and focus of the examination optical system 300 are performed by the drag operation and the pinch operation on the touch panel 75b, the examiner may also conduct an operation of optimizing the interference optical system 200 again. Upon receipt of an operation signal by the examiner, the control part 70 may optimize the interference optical system 200. The control part 70 may start photographing of the tomographic image 86 automatically after completion of optimization or start photographing of the tomographic image 86 after receipt of a signal representing the tap operation and others of the examiner.

When the focus of the fundus camera optical system 30 is adjusted by the multi-touch operation, the control part 70 may also automatically adjust focus of the interference optical system 200 based on a focus adjustment amount. This can save steps of manually adjusting respective focuses of the fundus camera optical system 30 and the interference optical system 200. When the focus of the interference optical system 200 is adjusted by the multi-touch operation, the focus of the fundus camera optical system 30 may also be adjusted together.

When the focus or the like of the interference optical system 200 or the fundus camera optical system is adjusted by the multi-touch operation, the multi-touch operation and others may be performed after it is enabled to be selected by touching on either image. Accordingly, it is possible to select the optical system to be adjusted by the multi-touch operation.

In the foregoing explanation, the XYZ drive part 6 drives the photographing part 3 to thereby adjust alignment between the examinee's eye and the photographing part 3, but the present disclosure is not limited thereto. For example, the XYZ drive part 6 may drive the examination optical system 300 contained in the photographing part 3 in the X-Y-Z directions to adjust alignment of the examination optical system 300 with respect to the examinee's eye.

The dragging direction on the touch panel and the moving direction of the photographing part 3 may be set arbitrarily. For instance, the control part 70 may switch between the operation mode of driving the photographing part 3 in an opposite direction to the direction in which the examiner drags his/her finger(s) as above and the operation mode of driving the photographing part 3 in the dragging direction.

The aforementioned structure uses the split targets as focus targets. As an alternative, a configuration of detecting an in-focus state by use of targets (e.g., a ring target, a point target) projected on the fundus may be utilized for focus detection. In this case, similar to the split target, the control part 70 may perform auto-focus control by bright spot analysis of the target(s).

The present example is explained in the ophthalmic apparatus by exemplifying the case of photographing a tomographic image of a fundus of an examinee's eye, but the present disclosure is not limited thereto. The technique of the present disclosure may also be applied to the case of photographing a tomographic image of an anterior segment of an examinee's eye.

The present example is configured with no operation member such as a joystick and a track ball, but it is not limited thereto. It may include an operation member such as a joystick and a track ball in addition to the touch panel.

## Claims

1. An ophthalmic apparatus (1) for examining an examinee's eye (E), comprising:
an optometry part (3) including an observation optical system (60) configured to observe an anterior segment of the examinee's eye and an examination optical system (30, 200, 300) configured to obtain examination information of the examinee's eye;
drive means (6, 49, 124a) configured to drive the optometry part in a direction of an examination optical axis with respect to the examinee's eye;
drive control means (70) configured to control driving of the drive means; and display means (75) including a touch panel (75b);
image output means (70) configured to output an anterior-segment image of the examinee's eye photographed by the observation optical system to the display means (75),
**characterized by**
further comprising detection means (70, 75b) configured to, in the display means on which the anterior-segment image output by the image output means is displayed, detect at least one of a change in relative distance between at least two designated points (Q1, Q2; R1, R2) designated on the the touch panel (75b) when the designated points are relatively moved and a rotating amount of at least two designated points (Q1, Q2; R1, R2) designated on the touch panel (75b), the touch panel being configured to allow designation of any position in an operational region set on a screen of the display means,
wherein the drive control means (70) is configured to perform drive control in which, when relative movement of at least the two designated points is detected by the detection means, the optometry part is driven in a direction of an optical axis based on at least one of the change in relative distance between and the rotating amount of at least the two designated points (Q1, Q2; R1, R2) detected by the detection means.

2. The ophthalmic apparatus according to claim 1, comprising first and second detection means (70, 75b), the detection means being the second detection means and the first detection means configured to, in the display means on which the anterior-segment image output by the image output means (70) is displayed, detect a moving direction and a moving amount of a designated point designated on the touch panel when the designated point is moved, the touch panel being configured to allow designation of any position in the operational region set on the screen of the display means,
wherein the drive control means (70) is configured to perform:
first drive control in which, when the first detection means detects movement of the designated point, the optometry part is driven in a perpendicular direction to an optical axis of the optometry part based on the moving direction and the moving amount of the designated point detected by the first detection means; and
second drive control in which, when the second detection means detects relative movement of at least the two designated points, the optometry part is driven in a direction of the optical axis based on at least one of the change in relative distance between and the rotating amount of at least the two designated points detected by the second detection means.

3. The ophthalmic apparatus according to claim 1 or 2, wherein the operational region is set on the anterior-segment image of the examinee's eye displayed on the display means.

4. The ophthalmic apparatus according to any one of claims 1 to 3, wherein
the observation optical system includes an imaging element having an imaging plane placed in a position conjugate with the anterior segment of the examinee's eye, the observation optical system being configured to project light on a fundus of the examinee's eye and photograph a transillumination image illuminated by fundus reflection light of the light projected on the fundus, and
the image output means is configured to output the transillumination image of the examinee's eye photographed by the observation optical system to the display means.

5. The ophthalmic apparatus according to any one of claims 1 to 4, wherein
the drive control means is configured to perform second drive control in which, based on the change in relative distance between at least the two designated points caused by a pinch operation on the touch panel and detected by the second detection means, driving of the drive part is controlled to move at least part of the examination optical system in an optical axis direction of the examination optical system.

6. The ophthalmic apparatus according to any one of claims 1 to 5, wherein
the examination optical system is an OCT optical system (200) configured to detect an interference signal by interference of measurement light scanned on the examinee's eye by scanning means and reference light to obtain a tomographic image (86), and
the drive part moves a first focus optical member (124) included in the OCT optical system in an optical axis direction.

7. The ophthalmic apparatus according to any one of claims 1 to 6, wherein
the examination optical system is an OCT optical system configured to detect an interference signal by interference of measurement light scanned on the examinee's eye by scanning means and reference light to obtain a tomographic image, and
the drive part moves a reference mirror (131) included in the OCT optical system in an optical axis direction.

8. The ophthalmic apparatus according to any one of claims 1 to 7, wherein
the examination optical system is a front photographing optical system (30) configured to receive reflection light from a fundus to obtain a front image of the fundus of the examinee's eye, and
the drive part moves a second focus optical member (32) included in the front photographing optical system in an optical axis direction.

9. The ophthalmic apparatus according to any one of claims 1 to 8, wherein
the drive control means is configured to perform the second drive control concurrently with the first drive control.

10. An ophthalmic apparatus control program to be used in an ophthalmic apparatus (1) comprising an optometry part (3) including an observation optical system (60) configured to observe an anterior segment of an examinee's eye (E) and an examination optical system (30, 200, 300) configured to obtain examination information of the examinee's eye, and drive means (6, 49, 124a) configured to three-dimensionally drive the optometry part with respect to the examinee's eye,
wherein the program will be executed by a processor of the ophthalmic apparatus to cause the ophthalmic apparatus to execute:
an image output step of outputting an anterior-segment image of the examinee's eye photographed by the observation optical system to display means (75),
**characterized in that**
the program will be executed by the processor of the ophthalmic apparatus to cause the ophthalmic apparatus to further execute:
a detection step of, in the display means on which the anterior-segment image output in the image output step is displayed, detecting at least one of a change in relative distance between at least two designated points (Q1, Q2; R1, R2) designated on a touch panel (75b) when the designated points are relatively moved and a rotating amount of at least two designated points (Q1, Q2; R1, R2) designated on a touch panel (75b), the touch panel being configured to allow designation of any position in an operational region set on a screen of the display control means; and
a drive step of driving the optometry part in a direction of the optical axis based on the change in relative distance between and a the amount of at least the two designated points detected (Q1, Q2; R1, R2) in the detection step when relative movement of at least the two designated points is detected in the detection step.

11. The ophthalmic apparatus according to any one of claims 1 to 9, wherein the anterior-segment image is a still image.

12. The ophthalmic apparatus according to any one of claims 2 to 9, wherein
the drive control means is configured to set a driving direction of the optometry part according to the moving direction of the designated point detected by the first detection means in the first drive control.

## Patentansprüche

1. Ophthalmische Vorrichtung (1) zum Untersuchen eines Auges (E) eines Patienten, aufweisend:
einen Optometrieteil (3) mit einem optischen Beobachtungssystem (60), das ausgestaltet ist, um ein vorderes Segment des Patientenauges zu überwachen, und einem optischen Untersuchungssystem (30, 200, 300), das ausgestaltet ist, um Untersuchungsinformationen des Patientenauges zu erhalten;
eine Antriebseinrichtung (6, 49, 124a), die ausgestaltet ist, um den Optometrieteil in einer Richtung einer optischen Untersuchungsachse relativ zum Patientenauge anzutreiben;
eine Antriebssteuereinrichtung (70), die ausgestaltet ist, um das Antreiben der Antriebseinrichtung zu steuern;
eine Anzeigeeinrichtung (75), die ein Touch Panel (75b) enthält; und
eine Bildausgabeeinrichtung (70), die ausgestaltet ist, um ein Bild des vorderen Segments des Patientenauges, das durch das optische Beobachtungssystem photographiert wurde, an die Anzeigeeinrichtung (75) auszugeben,
**gekennzeichnet durch**
weiteres Aufweisen einer Erfassungseinrichtung (70, 75b), die ausgestaltet ist, um in der Anzeigeeinrichtung, auf der das von der Bildausgabeeinrichtung ausgegebene Bild des vorderen Segments angezeigt wird, eine Veränderung des relativen Abstands zwischen wenigstens zwei gekennzeichneten Punkten (Q1, Q2; R1, R2), die auf dem Touch Panel (75b) gekennzeichnet sind, wenn sich die gekennzeichneten Punkte relativ zueinander beweg, und/oder ein Rotationsmaß von wenigstens zwei gekennzeichneten Punkten (Q1, Q2; R1, R2), die auf dem Touch Panel (75b) gekennzeichnet sind, zu erfassen, wobei das Touch Panel ausgestaltet ist, um eine Kennzeichnung einer beliebigen Position in einem Betriebsbereich zu ermöglichen, der auf einem Bildschirm der Anzeigeeinrichtung eingestellt wird,
wobei die Antriebssteuereinrichtung (70) ausgestaltet ist, um eine Antriebssteuerung durchzuführen, bei der, wenn eine relative Bewegung der wenigstens zwei gekennzeichneten Punkte durch die Erfassungseinrichtung erfasst wird, der Optometrieteil basierend auf der Veränderung des relativen Abstands zwischen den zwei gekennzeichneten Punkten (Q1, Q2; R1, R2) und/oder dem Rotationsmaß der wenigstens zwei gekennzeichneten Punkte (Q1, Q2; R1, R2), die durch die Erfassungseinrichtung erfasst wurden, in einer Richtung einer optischen Achse angetrieben wird.

2. Ophthalmische Vorrichtung nach Anspruch 1, aufweisend eine erste und eine zweite Erfassungseinrichtung (70, 75b), wobei die Erfassungseinrichtung die zweite Erfassungseinrichtung und die erste Erfassungseinrichtung ist, die ausgestaltet sind, um in der Anzeigeeinrichtung, auf der das von der Bildausgabeeinrichtung (70) ausgegebene Bild des vorderen Segments angezeigt wird, eine Bewegungsrichtung und ein Bewegungsmaß eines gekennzeichneten Punktes, der auf dem Touch Panel gekennzeichnet ist, zu erfassen, wenn sich der gekennzeichnete Punkt bewegt, wobei das Touch Panel ausgestaltet ist, um das Kennzeichnen einer beliebigen Position in dem auf dem Bildschirm der Anzeigeeinrichtung eingestellten Betriebsbereich zu erlauben,
wobei die Antriebssteuereinrichtung (70) ausgestaltet ist zum Durchführen
einer ersten Antriebssteuerung, bei der, wenn die erste Erfassungseinrichtung eine Bewegung des gekennzeichneten Punktes erfasst, der Optometrieteil basierend auf der Bewegungsrichtung und dem Bewegungsmaß, die durch die erste Erfassungseinrichtung erfasst werden, in einer senkrechten Richtung zu einer optischen Achse des Optometrieteils angetrieben wird; und
einer zweiten Antriebssteuerung, bei der, wenn die zweite Erfassungseinrichtung eine relative Bewegung der wenigstens zwei gekennzeichneten Punkte erfasst, der Optometrieteil basierend auf der Veränderung des relativen Abstands zwischen den wenigstens zwei gekennzeichneten Punkten und/oder dem Rotationsmaß der wenigstens zwei gekennzeichneten Punkte, die durch die zweite Erfassungseinrichtung erfasst werden, in einer Richtung der optischen Achse angetrieben wird.

3. Ophthalmische Vorrichtung nach Anspruch 1 oder 2, bei welcher der Betriebsbereich auf dem Bild des vorderen Segments des Patientenauges, das auf der Anzeigeeinrichtung angezeigt wird, eingestellt wird.

4. Ophthalmische Vorrichtung nach einem der Ansprüche 1 bis 3, bei welcher
das optische Beobachtungssystem ein bilderzeugendes Element mit einer Abbildungsebene, die in einer Position angeordnet ist, die mit dem vorderen Segment des Patientenauges verbunden ist, enthält, wobei das optische Beobachtungssystem ausgestaltet ist, um Licht auf einen Hintergrund des Patientenauges zu richten und ein durch Hintergrundreflexionslicht des auf den Hintergrund gerichteten Lichts erleuchteten Durchleuchtungsbildes zu photographieren, und die Bildausgabeeinrichtung ausgestaltet ist, um das durch das optische Beobachtungssystem photographierte Durchleuchtungsbild des Patientenauges an die Anzeigeeinrichtung auszugeben.

5. Ophthalmische Vorrichtung nach einem der Ansprüche 1 bis 4, bei welcher
die Antriebssteuereinrichtung ausgestaltet ist, um eine zweite Antriebssteuerung durchzuführen, bei der basierend auf der Veränderung des relativen Abstands zwischen den wenigstens zwei gekennzeichneten Punkten, die durch eine Druckbetätigung auf dem Touch Panel verursacht und durch die zweite Erfassungseinrichtung erfasst wird, ein Antreiben des Antriebsteils so gesteuert wird, dass wenigstens ein Teil des optischen Untersuchungssystems in einer Richtung der optischen Achse des optischen Untersuchungssystems bewegt wird.

6. Ophthalmische Vorrichtung nach einem der Ansprüche 1 bis 5, bei welcher
das optische Untersuchungssystem ein optisches OCT-System (200) ist, das ausgestaltet ist, um ein Interferenzsignal durch eine Interferenz von Messlicht, das auf dem Patientenauge durch eine Abtasteinrichtung abgetastet wird, und Referenzlicht zu erfassen, um ein tomographisches Bild (86) zu erhalten, und
das Antriebsteil ein erstes optisches Fokussierelement (124), das in dem optischen OCT-System enthalten ist, in einer Richtung der optischen Achse bewegt.

7. Ophthalmische Vorrichtung nach einem der Ansprüche 1 bis 6, bei welcher
das optische Untersuchungssystem ein optisches OCT-System ist, das ausgestaltet ist, um ein Interferenzsignal durch Interferenz von Messlicht, das auf dem Patientenauge durch eine Abtasteinrichtung abgetastet wird, und Referenzlicht zu erfassen, um ein tomographisches Bild zu erhalten, und
das Antriebsteil einen Referenzspiegel (131), der in dem optischen OCT-System enthalten ist, in einer Richtung der optischen Achse bewegt.

8. Ophthalmische Vorrichtung nach einem der Ansprüche 1 bis 7, bei welcher
das optische Untersuchungssystem ein optisches Frontalphotographiesystem (30) ist, das ausgestaltet ist, um Reflexionslicht von einem Hintergrund zu empfangen, um ein Frontalbild des Hintergrunds des Patientenauges zu erhalten,
und
das Antriebsteil ein zweites optisches Fokussierelement (32), das in dem optischen Frontalphotographiesystem enthalten ist, in einer Richtung der optischen Achse bewegt.

9. Ophthalmische Vorrichtung nach einem der Ansprüche 1 bis 8, bei welcher
die Antriebssteuereinrichtung ausgestaltet ist, um die zweite Antriebssteuerung gleichzeitig mit der ersten Antriebssteuerung durchzuführen.

10. Steuerprogramm für eine ophthalmische Vorrichtung zur Verwendung in einer ophthalmischen Vorrichtung (1) mit einem Optometrieteil (3), der ein optisches Beobachtungssystem (60), das ausgestaltet ist, um ein vorderes Segment eines Patientenauges (E) zu überwachen, und ein optisches Untersuchungssystem (30, 200, 300), das ausgestaltet ist, um Untersuchungsinformationen des Patientenauges zu erhalten, enthält, und einer Antriebseinrichtung (6, 49, 124a), die ausgestaltet ist, um den Optometrieteil relativ zum Patientenauge dreidimensional anzutreiben,
wobei das Programm durch einen Prozessor der ophthalmischen Vorrichtung ausgeführt wird, um die ophthalmische Vorrichtung ausführen zu lassen:
einen Bildausgabeschritt des Ausgebens eines Bildes eines vorderen Segments des Patientenauges, das durch das optische Beobachtungssystem photographiert wurde, an eine Anzeigeeinrichtung (75),
**dadurch gekennzeichnet, dass**
das Programm durch den Prozessor der ophthalmischen Vorrichtung ausgeführt wird, um die ophthalmische Vorrichtung ferner ausführen zu lassen:
einen Erfassungsschritt des Erfassens einer Veränderung des relativen Abstands zwischen wenigstens zwei gekennzeichneten Punkten (Q1, Q2; R1, R2), die auf einem Touch Panel (75b) gekennzeichnet sind, wenn sich die gekennzeichneten Punkte relativ zueinander bewegen, und/oder eines Rotationsmaßes von wenigstens zwei gekennzeichneten Punkten (Q1, Q2; R1, R2), die auf einem Touch Panel (75b) gekennzeichnet sind, in der Anzeigeeinrichtung, auf der das im Bildausgabeschritt ausgegebene Bild des vorderen Segments angezeigt wird, wobei das Touch Panel ausgestaltet ist, um eine Kennzeichnung einer beliebigen Position in einem Betriebsbereich zu ermöglichen, der auf einem Bildschirm der Anzeigesteuereinrichtung eingestellt wird; und
einen Antriebsschritt des Antreibens des Optometrieteils basierend auf der Veränderung des relativen Abstands zwischen den zwei gekennzeichneten Punkten (Q1, Q2; R1, R2) und/oder dem Rotationsmaß der wenigstens zwei gekennzeichneten Punkte (Q1, Q2; R1, R2), die im Erfassungsschritt erfasst wurden, in einer Richtung der optischen Achse, wenn im Erfassungsschritt eine relative Bewegung der wenigstens zwei gekennzeichneten Punkte erfasst wird.

11. Ophthalmoische Vorrichtung nach einem der Ansprüche 1 bis 9, bei welcher das Bild des vorderen Segments ein Standbild ist.

12. Ophthalmische Vorrichtung nach einem der Ansprüche 2 bis 9, bei welcher
die Antriebssteuereinrichtung ausgestaltet ist, um in der ersten Antriebssteuerung eine Antriebsrichtung des Optometrieteils gemäß der durch die erste Erfassungseinrichtung erfassten Bewegungsrichtung des gekennzeichneten Punktes einzustellen.

## Revendications

1. Appareil ophtalmique (1) pour examiner l'oeil d'un patient (E), comprenant :
une partie d'optométrie (3) comprenant un système optique d'observation (60) configuré pour observer un segment antérieur de l'oeil du patient et un système optique d'examen (30, 200, 300) configuré pour obtenir des informations sur l'examen de l'oeil du patient ;
un moyen d'entraînement (6, 49, 124a) configuré pour entraîner la partie d'optométrie dans une direction d'un axe optique d'examen par rapport à l'oeil du patient ;
un moyen de commande d'entraînement (70) configuré pour commander l'entraînement du moyen d'entraînement ; et
un moyen d'affichage (75) comprenant un écran tactile (75b) ;
un moyen de sortie d'image (70) configuré pour afficher une image de segment antérieur de l'oeil du patient photographié par le système optique d'observation sur le moyen d'affichage (75),
**caractérisé en ce que**
il comprend en outre un moyen de détection (70, 75b) configuré, dans le moyen d'affichage sur lequel l'image de segment antérieur sortie par le moyen de sortie d'image est affichée, pour détecter au moins un parmi un changement de distance relative entre au moins deux points désignés (Q1, Q2 ; R1, R2) désignés sur l'écran tactile (75b) lorsque les points désignés sont déplacés de manière relative et
une quantité de rotation d'au moins deux points désignés (Q1, Q2 ; R1, R2) désignés sur l'écran tactile (75b), l'écran tactile étant configuré pour permettre la désignation de n'importe quelle position dans une région opérationnelle réglée sur un écran du moyen d'affichage,
dans lequel le moyen de commande d'entraînement (70) est configuré pour effectuer une commande d'entraînement, dans laquelle, lorsque le déplacement relatif d'au moins les deux points désignés est détecté par le moyen de détection, la partie d'optométrie est entraînée dans une direction d'un axe optique sur la base d'au moins un parmi le changement de distance relative entre et la quantité de rotation d'au moins les deux points désignés (Q1, Q2 ; R1, R2) détectés par le moyen de détection.

2. Appareil ophtalmique selon la revendication 1, comprenant un premier et deuxième moyen de détection (70, 75b), le moyen de détection étant le deuxième moyen de détection et le premier moyen de détection configuré, dans le moyen d'affichage sur lequel l'image de segment antérieur sortie par le moyen de sortie d'image (70) est affichée, pour détecter une direction de déplacement et une quantité de déplacement d'un point désigné désigné sur l'écran tactile lorsque le point désigné est déplacé, l'écran tactile étant configuré pour permettre la désignation de n'importe quelle position dans la région opérationnelle réglée sur l'écran du moyen d'affichage,
dans lequel le moyen de commande d'entraînement (70) est configuré pour effectuer :
une première commande d'entraînement dans laquelle, lorsque le premier moyen de détection détecte un déplacement du point désigné, la partie d'optométrie est entraînée dans une direction perpendiculaire à un axe optique de la partie d'optométrie sur la base de la direction de déplacement et de la quantité de déplacement du point désigné détecté par le premier moyen de détection ; et
une deuxième commande d'entraînement dans laquelle, lorsque le deuxième moyen de détection détecte un déplacement relatif d'au moins les deux points désignés, la partie d'optométrie est entraînée dans une direction de l'axe optique sur la base d'au moins un parmi le changement de distance relative entre et la quantité de rotation d'au moins les deux points désignés détectés par le deuxième moyen de détection.

3. Appareil ophtalmique selon la revendication 1 ou 2, dans lequel la région opérationnelle est réglée sur l'image de segment antérieur de l'oeil du patient affichée sur le moyen d'affichage.

4. Appareil ophtalmique selon l'une quelconque des revendications 1 à 3, dans lequel
le système optique d'observation comprend un élément d'imagerie ayant un plan d'imagerie placé dans une position conjuguée au segment antérieur de l'oeil du patient, le système optique d'observation étant configuré pour projeter de la lumière sur un fond de l'oeil du patient et photographier une image de transillumination éclairée par la lumière de réflexion du fond d'oeil de la lumière projetée sur le fond d'oeil, et
le moyen de sortie d'image est configuré pour afficher l'image de transillumination de l'oeil du patient photographié par le système optique d'observation sur le moyen d'affichage.

5. Appareil ophtalmique selon l'une quelconque des revendications 1 à 4, dans lequel
le moyen de commande d'entraînement est configuré pour effectuer une deuxième commande d'entraînement dans laquelle, sur la base du changement de distance relative entre au moins les deux points désignés provoqué par une opération de pincement sur l'écran tactile et détecté par le deuxième moyen de détection, l'entraînement de la partie d'entraînement est commandé pour déplacer au moins une partie du système optique d'examen dans une direction d'axe optique du système optique d'examen.

6. Appareil ophtalmique selon l'une quelconque des revendications 1 à 5, dans lequel
le système optique d'examen est un système optique OCT (200) configuré pour détecter un signal d'interférence par interférence de lumière de mesure balayée sur l'oeil du patient par des moyens de balayage et de lumière de référence pour obtenir une image tomographique (86), et
la partie d'entraînement déplace un premier élément optique de focalisation (124) compris dans le système optique OCT dans une direction d'axe optique.

7. Appareil ophtalmique selon l'une quelconque des revendications 1 à 6, dans lequel
le système optique d'examen est un système optique OCT configuré pour détecter un signal d'interférence par interférence de lumière de mesure balayée sur l'oeil du patient par des moyens de balayage et de lumière de référence pour obtenir une image tomographique, et
la partie d'entraînement déplace un miroir de référence (131) compris dans le système optique OCT dans une direction d'axe optique.

8. Appareil ophtalmique selon l'une quelconque des revendications 1 à 7, dans lequel
le système optique d'examen est un système optique de photographie avant (30) configuré pour recevoir la lumière de réflexion d'un fond d'oeil pour obtenir une image avant du fond d'oeil du patient, et
la partie d'entraînement déplace un deuxième élément optique de focalisation (32) compris dans le système optique de photographie avant dans une direction d'axe optique.

9. Appareil ophtalmique selon l'une quelconque des revendications 1 à 8, dans lequel
le moyen de commande d'entraînement est configuré pour effectuer la deuxième commande d'entraînement en même temps que la première commande d'entraînement.

10. Programme de commande d'appareil ophtalmique destiné à être utilisé dans un appareil ophtalmique (1) comprenant une partie d'optométrie (3) comprenant un système optique d'observation (60) configuré pour observer un segment antérieur de l'oeil d'un patient (E) et un système optique d'examen (30, 200, 300) configuré pour obtenir des informations d'examen de l'oeil du patient, et un moyen d'entraînement (6, 49, 124a) configuré pour entraîner tridimensionnellement la partie d'optométrie par rapport à l'oeil du patient,
dans lequel le programme sera exécuté par un processeur de l'appareil ophtalmique pour amener l'appareil ophtalmique à exécuter :
une étape de sortie d'image consistant à afficher une image de segment antérieur de l'oeil du patient photographié par le système optique d'observation sur un moyen d'affichage (75),
**caractérisé en ce que**
le programme sera exécuté par le processeur de l'appareil ophtalmique pour amener l'appareil ophtalmique à exécuter en outre :
une étape de détection, dans le moyen d'affichage sur lequel l'image de segment antérieur sortie dans l'étape de sortie d'image est affichée, de détection d'au moins un parmi un changement de distance relative entre au moins deux points désignés (Q1, Q2 ; R1, R2) désignés sur un écran tactile (75b) lorsque les points désignés sont déplacés de manière relative et une quantité de rotation d'au moins deux points désignés (Q1, Q2 ; R1, R2) désignés sur un écran tactile (75b), l'écran tactile étant configuré pour permettre la désignation de n'importe quelle position dans une région opérationnelle réglée sur un écran du moyen de commande d'affichage ; et
une étape d'entraînement consistant à entraîner la partie d'optométrie dans une direction de l'axe optique sur la base du changement de distance relative entre et d'une la quantité d'au moins les deux points désignés détectés (Q1, Q2 ; R1, R2) dans l'étape de détection lorsqu'un déplacement relatif d'au moins les deux points désignés est détecté dans l'étape de détection.

11. Appareil ophtalmique selon l'une quelconque des revendications 1 à 9, dans lequel l'image de segment antérieur est une image fixe.

12. Appareil ophtalmique selon l'une quelconque des revendications 2 à 9, dans lequel
le moyen de commande d'entraînement est configuré pour régler une direction d'entraînement de la partie d'optométrie en fonction de la direction de déplacement du point désigné détecté par le premier moyen de détection dans la première commande d'entraînement.
